(19) Europäisches Patentamt
European Patent Office
Office européen des brevets

(11) EP 4 477 687 B1

(12) EUROPEAN PATENT SPECIFICATION

(45) Date of publication and mention of the grant of the patent:
**17.12.2025 Bulletin 2025/51**

(21) Application number: **23179196.3**

(22) Date of filing: **14.06.2023**

(51) International Patent Classification (IPC):
***C08G 65/22*** $^{(2006.01)}$ ***A61K 47/50*** $^{(2017.01)}$
***A61K 47/60*** $^{(2017.01)}$

(52) Cooperative Patent Classification (CPC):
**C08G 65/22; A61K 47/60**

# (54) IMMUNOLOGICALLY INACTIVE POLY(ETHYLENE GLYCOL) POLYMER DERIVATIVES AND BIOCONJUGATES THEREOF FOR USE AS ALTERNATIVE IN PEGYLATED THERAPEUTICS, PROCESSES FOR THEIR PREPARATION AND THEIR USE

IMMUNOLOGISCH INAKTIVE POLY(ETHYLENGLYCOL)-POLYMERDERIVATE UND BIOKONJUGATE DAVON ZUR VERWENDUNG ALS ALTERNATIVE IN PEGYLIERTEN THERAPEUTIKA, VERFAHREN ZU IHRER HERSTELLUNG UND IHRE VERWENDUNG

DÉRIVÉS DE POLYMÈRES DE POLY(ÉTHYLÈNE GLYCOL) IMMUNOLOGIQUEMENT INACTIFS ET LEURS BIOCONJUGUÉS POUR LEUR UTILISATION COMME ALTERNATIVE DANS DES MÉDICAMENTS PÉGYLÉS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**18.12.2024 Bulletin 2024/51**

(73) Proprietor: **Johannes Gutenberg-Universität Mainz**
**55122 Mainz (DE)**

(72) Inventors:
- **Frey, Holger**
  **55128 Mainz (DE)**
- **Matthes, Rebecca**
  **55128 Mainz (DE)**
- **Dreier, Philip**
  **55128 Mainz (DE)**
- **Fuß, Fabian**
  **55122 Mainz (DE)**

(74) Representative: **Durm Patentanwälte PartG mbB**
**Patentanwälte**
**Moltkestrasse 45**
**76133 Karlsruhe (DE)**

(56) References cited:
**WO-A1-2022/238532 US-A- 6 162 563**

- **ISONO TAKUYA ET AL: "Design and synthesis of thermoresponsive aliphatic polyethers with a tunable phase transition temperature", POLYMER CHEMISTRY, vol. 8, no. 37, 1 January 2017 (2017-01-01), Cambridge, pages 5698 - 5707, XP093108035, ISSN: 1759-9954, DOI: 10.1039/C7PY01238A**
- **SAYURI AOKI ET AL: "Novel Thermosensitive Polyethers Prepared by Anionic Ring-Opening Polymerization of Glycidyl Ether Derivatives", vol. 31, no. 11, 1 January 2002 (2002-01-01), pages 1128 - 1129, XP008158620, ISSN: 0366-7022, Retrieved from the Internet <URL:https://www.jstage.jst.go.jp/article/cl/31/11/31_11_1128/_article> [retrieved on 20021105], DOI: 10.1246/CL.2002.1128**
- **FERRIER R. C. ET AL: "Four-fold increase in epoxide polymerization rate with change of alkyl-substitution on mono-[mu]-oxo-dialuminum initiators", POLYMER CHEMISTRY, vol. 8, no. 31, 1 January 2017 (2017-01-01), Cambridge, pages 4503 - 4511, XP093108037, ISSN: 1759-9954, DOI: 10.1039/C7PY00894E**
- **ZHENG TAO ET AL: "A new branched copolyether-based polymer electrolyte for lithium batteries", SOLID STATE IONICS, vol. 259, 3 March 2014 (2014-03-03), pages 9 - 13, XP028841726, ISSN: 0167-2738, DOI: 10.1016/J.SSI.2014.02.011**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Introduction

The present invention concerns immunological inactive polyether polymers represented by the following formula [I]:

$$-[CH_2\text{-}CHR\text{-}O]_n\text{-} \qquad (I),$$

wherein at least 3 % of the residues R comprise ethylene oxide side chains, oligo(ethylene oxide) side chains and/or glycerol ether side chains and R may also be hydrogen or a C1 to C3-alkyloxymethyl side chain. The invention further concerns a process for the preparation of the polymers. Finally, the invention concerns the use of the polymers for the preparation of conjugates, especially bioconjugates and the conjugates themselves as alternative for PEGylated therapeutics. The conjugates may comprise bioactive compounds and/or lipids. They are especially useful for the preparation of vaccines, lipid particles, especially lipid nanoparticles and may be used for the preparation of vaccines against COVID-19.

### Background of the invention

The aliphatic polyether poly(ethylene glycol) (PEG) is the most widely employed polymer in pharmaceutics, medicine and drug delivery. PEGylation, i.e. the attachment of PEG to peptide drugs, was introduced in the 1970ies and has since then led to numerous highly efficient drugs. PEGylation is a form of conjugation, i.e. the attachment of a polymer to a molecule by a covalent bond. The main effect of the attachment of PEG chains is the so-called "stealth effect", which prevents close approach of and recognition by the immune system and subsequent binding and removal by the reticuloendothelial system (RES) in the liver. More simply, PEG attachment also prevents opsonization by proteins, when attached to the surfaces of nanocarriers for drug delivery. The inhibition of protein adsorption by PEG is further emphasized by fulfillment of the rules for protein-repellent surfaces derived by Whitesides (Yarovsky et al., "Quantitative design rules for protein-resistant surface coatings using machine learning", Scientific Rep. 2019, 9, 265). PEGylation is also used for "stealth liposomes" with long circulation times, low molecular weight drugs as well as for lipid nanoparticles. Such structures are employed for the transport and delivery of mRNA. Here the respective PEG-based lipids play a key role for the vaccination against the COVID-19 pandemic, enabling transport of mRNA to cells, avoiding undesired degradation processes. The recently approved mRNA vaccines from Moderna and Pfizer-BioNTech require PEGylated lipids to ensure the stability of the mRNA in the lipid-based nanoparticle. PEGylation also prevents coalescence of liposomes and similar structures in aqueous systems. Commonly, molecular weights in the range of 2 to 40 kg/mol are preferred for the PEGylation of drugs or nanocarriers. To sum up, PEG has become a standard for water-soluble medical polymers for a vast variety of medical and pharmaceutical uses, particularly in the therapy of cancer, chronic diseases and recently also for RNA delivery vehicles. The success of PEG may have had several reasons. Among them are commercial availability in sufficient quality and in a wide range of molar masses from the anionic ring-opening polymerization of ethylene oxide as well as its extremely low toxicity and high biocompatibility. Further, PEG can be prepared in narrow molecular weight distributions.

However, an increasing number of studies summarize concerns related to the presence of so-called anti-PEG antibodies (APAs) that are present in a large and constantly growing part of the population. Such antibodies can lead to accelerated clearance of PEGylated drugs from the blood stream, i.e. loss of the stealth effect that is crucial for therapeutic success, allergic reactions and in extreme and rare cases even potentially fatal anaphylactic shock. This undesired effect is observed both for PEGylated peptide drugs, PEGylated liposomes and other PEGylated nanocarriers as well as PEGylated small molecule drugs. Anti-PEG antibodies are also problematic with respect to PEGylated lipids for RNA-transporting lipid nanoparticles, e.g. in the context of COVID-19 vaccines. PEG-antibodies are often mentioned in the context of potential PEG alternatives as a motivation to look beyond PEG for alternatives. As an estimate, approximately 10% of the population experience allergic reactions when treated with PEGylated formulations. Only recently, the nature of the binding between PEG and anti-PEG antibodies has been studied by Lai et al (Lai et al, "Structure of an anti-PEG antibody reveals an open ring that captures highly flexible PEG polymers." Commun Chem 2020, 3, 124). They concluded that binding of PEG is due to an open ring structure of PEG captured by the APAs. By counting the number of monomer repeats of the PEG polymer interacting with the interior and exterior paratope of the Fab, they found the size of the PEG antigen epitope to be ~700 g/mol, equivalent to 16 monomer subunits. Thus, at least 16 ethylene glycol units are required for binding by the APA. Other works suggest that also shorter regular chain segments of PEG as well as the methoxy end group of mPEG ($\alpha$ -methoxy poly(ethylene glycol)) structure play a key role for the recognition. US 8,129,330 B2 therefore discloses methods for the preparation of PEG conjugates that are based on end group modification of the PEG chains. Variation of the end group of PEG is disclosed, aiming at minimizing interaction of for example methoxy end groups with

anti-PEG antibodies. The authors disclose conjugates, wherein a hydroxyl group is present on all of the distal polyalkylene glycol termini in said pure conjugate, and wherein said conjugate exhibits reduced antigenicity compared to mPEG-protein conjugates. It has been reported that antibodies elicited by PEG-OH have similar affinity to both mPEG and PEG-OH, while antibodies induced by mPEG recognize mPEG more effectively than PEG-OH (Sherman et al, "Role of the methoxy group in immune responses to mPEG-protein conjugates." Bioconjug Chem 2012, 23(3), 485-499; Sherman et al, "Selectivity of binding of PEGs and PEG-like oligomers to anti-PEG antibodies induced by methoxyPEG-proteins." Mol Immunol 2014, 57(2), 236-246.). These results have been obtained by using competitive ELISA and imply that the anti-PEG antibodies elicited by PEG-OH-proteins are directed against the backbone of the PEG (backbone-specific), while antibodies induced by mPEG-protein conjugates are methoxy group specific. It can be concluded that regular segments of at least 4-5 to 16 regularly arranged ethylene glycol units are required to achieve recognition and immunogenic reaction by anti-PEG antibodies. Recent clinical works demonstrated that the presence of antibodies against PEG permits to predict Pegasparagase allergic reactions and failure of rechallenge, emphasizing the clinical importance of anti-PEG antibodies for the success of the treatment of leukemia in this case (Liu et al, "Antibodies Predict Pegasparagase Allergic Reactions and Failure of Rechallenge", J. Clin. Oncol. 2019, 37, 2051).

Four strategies for the modification of the PEG structure have been developed to counteract undesired APA interaction of PEGylated therapeutics. Replacing the methoxy end group of mPEG by a hydroxyl group led to a strong decrease in the affinity for anti-PEG antibodies. However, it is obvious that for large scale commercial applications the presence of a hydroxyl group at the terminus would require protected functional initiators, which complicates existing synthetic protocols. A second recent strategy is disclosed in US 2019/0015520 A1 and relies on PEG-bottlebrush structures, i.e. small PEG structures appended to a poly(hydroxyethyl methacrylate) backbone. The inventors state that this type of PEG-architecture minimizes anti-PEG antigenicity, while preserving the desired stealth properties for surface coating. However, the resulting PEG-grafted copolymers exhibit rather broad, multimodal molecular weight distributions, which represent a major obstacle for approval for medical application and PEGylation analogous bioconjugation with peptides or lipids. Narrow distributions (Mw/Mn < 1.15) were exclusively feasible by performing a cost- and time-consuming preparative size-exclusion chromatography (SEC).

Another strategy is to exchange PEG with the linear polyether polyglycerol to increase the blood circulation time of liposomes and to avoid accelerated blood clearance (see Abu Lila et al, "Use of polyglycerol (PG), instead of polyethylene glycol (PEG), prevents induction of the accelerated blood clearance phenomenon against long-circulating liposomes upon repeated administration", International Journal of Pharmaceutics, 456 (2013) 235-242). However, the utilization of polyglycerol also results in the introduction of multiple hydroxyl functionalities to the liposome, which is in contrast to the aforementioned rules of Whitesides resulting in complex unspecific recognitions of peptides in the blood stream. Therefore, a targeted use of the conjugated drug or liposomes is not possible with polyglycerol.

A fourth strategy has been presented by the inventors of the present patent application, in which C1 to C3 alkoxymethyl side chains are appended to the backbone of the polymer (see WO 2022/238532 A1 and European patent application No. EP 22 203 748; not yet published). While this is an effective strategy, it has some limitations. Poly(ethylene oxides) with methoxymethyl side chains, e.g.

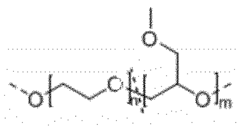

have a hydrophilicity that is practically identical to polyethylene glycol, but the side chains have limited spatial requirements. In poly(ethylene oxides) with C2 and C3 alkoxymethyl side chains, e.g.

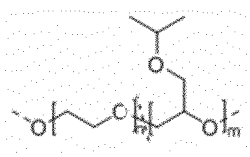

the side chains are more sterically demanding, but the polymers are less hydrophilic. Hydrophilicity is necessary to provide solubility and stealth effect to the polymers. A high steric footprint of the side chains is necessary to prevent crystallization and immunogenic reactions. Increased spatial requirements of the said PEG copolymers impede or disable interaction with anti-PEG antibodies according to the specific "lock and key principle". Equally important, the use of a PEGylated pharmaceutical agent has shown that the immunogenic system is still not very well understood. It seems that almost any chemical structure may trigger an immunological reaction. There is a need for a variety of tools for the manufacturing PEGylated drugs to choose from. Availability of a variety of polymers suitable for the PEGylation of drugs also provides the

opportunity to adapt the polymer to the structure of the physiologically active agent and may thus allow easier production, better delivery and/or improved effect of the drug.

Since PEGylated therapeutics are often used for chronic or in many cases otherwise fatal diseases, like in cancer therapy, in many cases repeated doses have to be administered over prolonged periods of time. PEG is a non-biodegradable polymer and has to be eliminated from the body through the kidneys. In studies on the renal filtration of PEGylated drugs, an undesirable accumulation of PEG in the kidneys and the associated pathological changes in the renal system could be demonstrated (see e.g. Bendele, A.; Seely, J.; Richey, C.; Sennello, G.; Shopp, G. Short Communication: Renal Tubular Vacuolation in Animals Treated with Polyethylene-Glycol-Conjugated Proteins. Toxicol. Sei. 1998, 42, 152-157). The tendency of polyethylene to crystallize can therefore lead to deposits in the kidney. The elimination through the kidneys also limits the molecular weight of PEG for medical therapeutics to the renal cutoff size, which for globular proteins is approximately 50 to 60 kDa.

The present inventors have reported in 2014 on the copolymerization of ethylene oxide with glycidyl methyl ether (GME) by use of the monomer activation method and tetraoctylammonium bromide and tris-*iso*-butylaluminum as initiators (see Frey et al, "A Challenging Comonomer Pair: Copolymerization of Ethylene Oxide and Glycidyl Methyl Ether to Thermoresponsive Polyethers", Macromolecules 2014, 47, 5492-5500). Rather broad molecular weight distributions were obtained (dispersity in the range of 1.21 to 1.5), which are clearly not suitable for medical applications. As discussed at the time, with the conventional anionic ring-opening method only molecular weights up to 3000 g/mol and high dispersities have been reported for the polymerization of GME. In previous studies regarding the homopolymerization of GME to poly(glycidyl methyl ether) (PGME) it was generally stated that polymerization causes difficulties, and no molecular weight control can be achieved by anionic ring-opening polymerization as employed for medical PEG (Frey et al., Biomacromolecules 2014, 15, 1935-1954). In all available reports regarding GME polymerization to date, either phosphazene bases were used as a catalyst or aluminum compounds had to be added in the way of a "monomer activated polymerization". The same applies to the reaction as published by Labbé et al. (Macromol. Symp. 2007, 249-250, 392-397) using monomer-activated ring-opening polymerization for synthesis of polyglycidylether. This technique suffers from a common side reaction described in figure 11. The undesired transfer of hydride leads to further initiating species, therefore limiting the required high end-group fidelity. Additionally, the occurrence of initiation by iso-propyl via monomer-activated ring-opening polymerization is also reported in the literature e.g. in Billouard, C.; Carlotti, S.; Desbois, P.; Deffieux, A. "Controlled" High-Speed Anionic Polymerization of Propylene Oxide Initiated by Alkali Metal Alkoxide/Trialkylaluminum Systems. Macromolecules 2004, 37 (11), 4038-4043. DOI: 10.1021/ma035768t and Herzberger, J.; Niederer, K.; Pohlit, H.; Seiwert, J.; Worm, M.; Wurm, F. R.; Frey, H. Polymerization of Ethylene Oxide, Propylene Oxide, and Other Alkylene Oxides: Synthesis, Novel Polymer Architectures, and Bioconjugation. Chemical reviews 2016, 116 (4), 2170-2243. DOI: 10.1021/acs.chemrev.5b00441. None of these polymers or copolymers are suitable for medical applications for several reasons: (i) traces of phosphazene bases lead to toxicity and cannot be removed; (ii) dispersities exceed 1.1, which is prohibitive for most medical applications and (iii) end-group fidelity of the polymers derived from the methods is insufficient for attachment to peptides, drugs or lipids via bioconjugation. Therefore, neither PGME homopolymer nor copolymers of GME have been considered for PEGylation type applications to date.

The formation and retention of well-defined polymer end-groups (end-group fidelity) is a critical tool for chain extension and all post polymerization reactions. End group fidelity is also a decisive factor for the use of polymers in bioconjugates for pharmaceutical applications, in which the biomolecules are connected by means of the end-groups. Polymers with low end-group fidelity comprise a large amount of macromolecules that are not bound to the biomolecule and cannot be removed from the mixture without undue expenditure. The formed conjugates do not have the required purity. The loss of end-groups is therefore not desirable but cannot be completely avoided. End-group fidelity varies greatly depending on the reaction conditions in the preparation of the polymers.

Polyether copolymers having a main chain derived from ethylene oxide and an oligooxyethylene side chain are known in the art. They are usually used in applications for solid electrolytes, for example in batteries. Most of the polymers described have high or very high molecular weights and a high dispersity. US 6,162,563 A may be mentioned as example, which discloses such polymers. In the working examples polymers with molecular weights above 1,000,000 g/mol are prepared. The dispersities are not disclosed, but a heterogenous catalyst comprising tributyl tin chloride and tributyl phosphate is used. The polymers therefore have high dispersities.

Isono Takuya et al, "Design and synthesis of thermoresponsive aliphatic polyethers with a tunable phase transition temperature", Polymer Chemistry, Vol. 8, no. 37,1 January 2017, pages 5698-5707 discloses design and synthesis of thermoresponsive poly(glycidyl ether) homopolymers and copolymers with varying side chain structure, molecular weight and main chain tacticity and their thermoresponsive properties. US 6 162 563 A discloses solids electrolytes comprising copolymers of ethylene oxide and glycidyl ethers of $-(CH_2-CH_2-O)_n-R^1$ with $R^1$ = alkyl with 1 to 12 carbon atoms.

## Purpose of the invention

It is the objective of the present invention to provide alternative polymers that are able to replace PEG in its applications and that can alleviate at least some of the aforementioned disadvantages of PEG. The alternative polymer should especially be suited to replace PEG in pharmaceutical applications. They should have low tendency to crystallize. It is further an objective to provide polymers with low dispersity. A further objective is to provide polymers with high end-group fidelity. It is also desired to provide a process that allows preparation of such polymers in a simple and commercially feasible way. The alternative polymers should have a low affinity towards anti-PEG antibodies (APAs). A further goal is to provide polymers with a low immunogenicity.

## Detailed description

The present invention is as described in the appended claims. The present invention concerns polyether polymers comprising monomer units that have a structure of formula (I):

$$-[CH_2\text{-}CHR\text{-}O]_n- \qquad (I),$$

wherein residues R are selected independently from each other from the group consisting of hydrogen, $R^1$ and $R^2$; wherein residues $R^1$ are selected independently of each other from the group consisting of

$$-CH_2\text{-}O\text{-}(CH_2\text{-}CH_2\text{-}O)_m\text{-}R^3,$$

$$-CH_2\text{-}O\text{-}(CH_2\text{-}CH_2\text{-}O)_x\text{-}CH(CH_2\text{-}O\text{-}(CH_2\text{-}CH_2\text{-}O)_a\text{-}R^3)(CH_2\text{-}O\text{-}(CH_2\text{-}CH_2\text{-}O)_b\text{-}R^3)\ ,$$

$$-CH_2\text{-}O\text{-}(CH_2\text{-}CH_2\text{-}O)_y\text{-}CH_2\text{-}CH(O\text{-}(CH_2\text{-}CH_2\text{-}O)_c\text{-}R^3)\text{-}CH_2\text{-}O\text{-}(CH_2\text{-}CH_2\text{-}O)_d\text{-}R^3,$$

wherein m are independently of each other 1, 2, 3, 4 or 5 and a, b, c, d, x and y, are independently from each other 0 or 1;
wherein $R^2$ is $-CH_2\text{-}O\text{-}R^3$,
wherein residues $R^3$ are selected independently of each other from the group consisting of hydrogen, methyl, ethyl, *n*-propyl and *iso*-propyl;
and wherein at least 3 % of the residues R are residues $R^1$, based on the total number of all residues R in the polymer;
wherein the polyether polymers of formula (I) are either homopolymers or statistical copolymers and wherein at least 97 % of the monomer units of the polyether polymers are of formula (I), based on all monomer units of the polyether polymers;
wherein the polyether polymers have at least a number average molecular weight in the range of 1,000 to 50,000 g/mol as determined by MALDI-TOF MS and the dispersity of the polyether polymers is 1.15 or less as measured by size exclusion chromatography in DMF by calibration with PEG standards characterized in that the polyether polymers of Formula (I) are represented by the following formula [II]:

$$X\text{-}[CH_2CHR\text{-}O]_n\text{-}CH_2CHR\text{-}Y \qquad [II]$$

wherein R is as defined as in formula (I)
wherein X is selected from the group consisting of hydroxyl or -O-X',
wherein X' is selected from the group consisting of

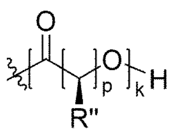
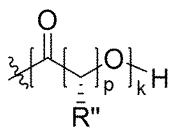
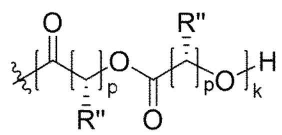

wherein R" = H or alkyl, alkenyl, aryl, alkoxymethyl, alkenyloxymethyl, aryloxymethyl, aminomethyl and thioalkoxymethyl and
p = 1, 2, 3, 4 or 5, and
k = 5-500;

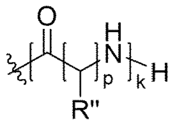 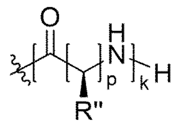 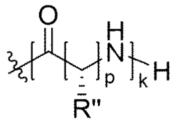

wherein R" = H or alkyl, alkenyl, aryl, alkoxymethyl, alkenyloxymethyl, aryloxymethyl, aminomethyl and thioalkoxymethyl, and
p = 1, 2, 3, 4 or 5 and
k = 5-500; and

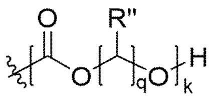

wherein R" = H or alkyl, alkenyl, aryl, alkoxymethyl, alkenyloxymethyl, aryloxymethyl, aminomethyl and thioalkoxymethyl
q = 3, 4 or 5 and
k = 5-500;
and

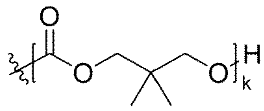

wherein k = 5-500; and
wherein Y is -Z-W, wherein
Z is -O-R'-O- and
W is $-CH_2CHR-(O-CH_2CHR-)_{n'}-V$;
wherein V is X or X';
wherein R' is selected from the group consisting of

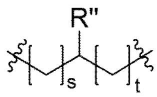

wherein
R" = H or alkyl, alkenyl, aryl, alkoxymethyl, alkenyloxymethyl, aryloxymethyl, aminomethyl and thioalkoxymethyl, and
s = 0-20
t = 0-20; and
further R' is selected from

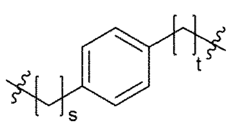
s = 0-20
t = 0-20

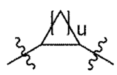
u = 1-10

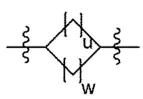
u = 1-10
w = 1-10

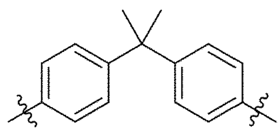
.

n may be any integer, as long as the molecular weight requirements are met. It is preferably an integer in the range of 10 to 1000. These new polyether polymers are a new type of material, which behaves very different to PEG in regard to the affinity towards anti-PEG antibodies (APAs) and have a low immunogenicity. They also have a low crystallinity. But the polyether polymers of the present invention behave very similar to PEG with respect to various features that are important for pharmaceutical applications, including aqueous solubility, hydration, cell viability and biocompatibility. In the present patent application polymers of the present invention are also addressed as "scPEG" (poly(ethylene oxide) derivatives with side chains) and the corresponding monomers

with side chains are also addressed as "scEO".

Key to the reduced interaction with APAs is the use of substituents on the PEG backbone, i.e. side chains. The increased spatial requirements of the said PEG copolymers impede or disable interaction with anti-PEG antibodies according to the specific "lock and key principle". Besides the steric impact, the random or statistical distribution of the substituents over the polymer backbone and/or the random or statistical steric orientation of the substituents furthermore impede the development and formation of specific anti-polymer antibodies.

Key to the similarity of the features of PEG and the inventive polymers is the use of alkoxymethyl side chains, which are structurally close to PEG. This maintains water solubility and other properties in view of PEG. A repeating group of the present polymer wherein R comprises an oligooxyethylene side chain with a methoxy end group, e.g. $-CH_2-O-(CH_2-CH_2-O)_m-CH_3$ has the same molecular formula as the same amount of repeating units of PEG $(-CH_2-CH_2-O)_{m+1}-$ and is therefore a constitutional isomer of m+1 main chain repeating units of PEG. It comprises the same number of atoms and very similar functional groups. This leads to similar properties, especially to similar water solubility, which is important for applications in biological systems. The steric hindrance provides low crystallinity. The same is true for all other side chains R of the polymers of the present invention including the side chains with a glycerol ether partial structure.

Methoxymethyl, Ethoxymethyl, n-propoxymethyl and iso-propoxymethyl may be used as residue R or $R^2$, respectively, to adjust the properties of the polymers further, for example their solubility, interaction with lipids, properties in respect to the passage of biological membranes etc. However, Ethoxymethyl, n-propoxymethyl and iso-propoxymethyl lead to a reduced solubility in water. The choice of such substituents also leads to a deviation from the behavior of PEG for other properties. For this reason, the amount of repeating units with the residues selected from the group consisting of ethoxymethyl, n-propoxymethyl and iso-propoxymethyl is preferably limited to 50% of all residues R of the polymer of formula (I), preferably to 30%, more preferably to 10 % and most preferably to 1%. Also in embodiments of the present invention in which at least 10% of residue R of the polymers are selected from the group consisting of ethoxymethyl, n-propoxymethyl and iso-propoxymethyl at least 10 % are hydrogen or $R^1$ and more preferred at least 25 % are hydrogen or $R^1$. In a further preferred embodiment only up to 20% of the residues R may be selected from the group consisting of ethoxymethyl, n-propoxymethyl and iso-propoxymethyl. These residues are not required for some of the uses of the polymers of the present invention in pharmaceutical applications. For this reason, polymers of the present invention are preferred where R is selected from the group consisting of hydrogen, $R^1$ and methoxymethyl. Obviously, ethylene oxide repeating units in the inventive polymers also lead to similar properties with PEG. This selection therefore provides the polymers that are closest in behavior to PEG.

Higher homologues of methoxymethyl, i.e. where $R^2$ is C4 alkoxymethyl and higher lead to increasingly low solubility in water and other detrimental properties and should therefore generally be avoided. They may however be used in small quantities when required, preferably in amounts of 5 % or less and more preferably in amounts of 3 % or less and most preferred 1 % or less, based on the sum of all residues R. If the polyether of the present invention comprises higher homologues of methoxymethyl, where R is C4-alkoxymethyl or higher, the same proviso for the presence of hydrogen applies with the same preferred amounts. Various other comonomers may be also used in small amounts, i.e. preferably less than 3 wt%, more preferably less than 1 wt% and most preferred in less than 0.1 wt% based on the number average molecular weight of the polymer of formula [I], if necessary. They may for example be used to introduce functional groups or side chains into the polymer. Best suited for the use in pharmaceutical application are however usually polymers of the present invention that comprise only the repeating units of formula (I).

As mentioned before, ethylene oxide repeating units and glycidyl ether repeating units with ether side chains, provide polymers with very similar properties to poly(ethylene oxide). Therefore even homopolymers of glycidyl ether repeating units with ether side chains may be used in the conjugates of the present invention. This is however not preferred. The properties of the polymers of the present invention are still closer to the properties of PEG, when at least some of the residues R are selected to be hydrogen. Furthermore, immunogenicity of copolymers will be reduced as compared to homopolymers, because they do not only differ in the orientation of side groups, but also in the incorporation of side groups along the polymer backbone. The polymers of the present invention are copolymers.

Hydroxyl groups in the side chains may be used to further modify the polymer or may increase interaction with biological systems. Where this is preferred, hydroxyl groups may be part of the polymers, as defined above. However, if it is desired to reduce interactions with biological systems, the amount of hydroxyl groups should be limited. Preferred are polymers of the present invention where no more than 10%, more preferably no more than 1%, even more preferably 0.1% and most preferred none of the rests R comprise hydroxyl groups, i.e. residues $R^3$ are selected independently of each other from the group consisting of methyl, ethyl, n-propyl and iso-propyl. Furthermore, the aforementioned considerations for the selection of the residue $R^2$ apply mutatis mutandis to the residue $R^3$. Therefore, the amount of repeating units in which $R^3$ is selected from the group consisting of ethyl, n-propyl and iso-propyl is preferably limited to 50% of all residues R of the polymer of formula (I), preferably to 30%, more preferably to 10 % and most preferably to 1%. Also in embodiments of the present invention in which at least 10% of residue $R^3$ of the polymers are selected from the group consisting of ethyl, n-propyl and iso-propyl preferably at least 10 % are hydrogen and more preferred at least 25 % are hydrogen. In a further preferred embodiment only up to 20% of the residues $R^3$ may be selected from the group consisting of ethyl, n-propyl and iso-propyl. Most preferred are polymers of the present invention where all residues $R^3$ are methyl. These polymers provide the polymers that are closest in behavior to PEG.

The largest differences between the side groups and therefore the lowest immunogenicity is provided by polymers in which some of the repeating units have no side groups, i.e. where R is hydrogen. Preferred are therefore polyether polymers of the present invention wherein at least 3 % of the residues R are hydrogen. More preferred at least 10% of residues R are hydrogen, even more preferred at least 30% of the residues R are hydrogen and most preferred, at least 50% of the residues R are hydrogen.

For some application polyether polymers are preferred in which some of the rests R are $R^2$. This may for example be used to adjust the hydrophilicity, crystallinity or other features of the polyether polymers of the present invention. In such a case polyether polymers of the present invention wherein at least 3 % of residues R are residues $R^2$ may be used. Preferably the amount of residue $R^2$ is in the range of 3 to 50%, more preferably 5 to 20% and even more preferably 5 to 10%.

As discussed above, the properties of the polymers of the present invention are most pronounced, if the differences between the monomer units of the backbone are greatest. A combination of monomers where R is hydrogen and $R^1$ is therefore preferred. Preferably 3 to 95% of the residues R are hydrogen and 3 to 95% of the residues R are $R^1$. More preferred are 10 to 90% of the residues R are hydrogen and 10 to 90% of the residues R are $R^1$. Even more preferred are 20 to 80% of the residues R are hydrogen and 20 to 80% of the residues R are $R^1$. The lowest immunogenicity are providing polymers of the present invention, where about 50 % of all residues R are hydrogen. For this reason polymers are preferred where 30 to 70% of the residues R are hydrogen and 30 to 70% of the residues R are R1. Especially useful in pharmaceutical applications is a polyether polymer of the present invention, wherein the residue R is selected from the group consisting of hydrogen and $R^1$. The aforementioned preferred ranges apply accordingly also to embodiments where R is selected from the group consisting of hydrogen and $R^1$.

As mentioned above, the present polymers may comprise other monomer units than those of formula (I). However, for pharmaceutical applications as far as possible, a homogeneous composition may be preferred. It is therefore preferred that the polyether polymers of the present invention comprise at least 80% of monomer units that have the structure of formula (I). More preferred are at least 90%, even more preferred at least 98% and most preferred consist the polyether polymers of the present invention of monomer units of formula (I).

In one embodiment, the polyether polymers of the present invention comprise at least 90% of monomer units that have the structure of formula (I) and 10 to 90 % of the residues R are hydrogen and 10 to 90% of the residues R are $R^1$.

In one embodiment, the polyether polymers of the present invention comprise at least 95% of monomer units that have the structure of formula (I) and 50 to 90 % of the residues R are hydrogen and 10 to 50% of the residues R are $R^1$.

In one embodiment, all monomer units of the polyether polymers of the present invention have the structure of formula (I) and 60 to 82 % of the residues R are hydrogen and 18 to 40% of the residues R are $R^1$.

In one embodiment, the polyether polymers of the present invention comprise at least 90% of monomer units that have the structure of formula (I) and 10 to 90 % of the residues R are hydrogen and 10 to 90% of the residues R are $R^1$ and the polyether polymers of the present invention comprises less than 20 % of rests R that are $R^2$.

In one embodiment, the polyether polymers of the present invention comprise at least 95% of monomer units that have the structure of formula (I) and 50 to 90 % of the residues R are hydrogen and 10 to 50% of the residues R are $R^1$ and the polyether polymers of the present invention comprises less than 10 % of rests R that are $R^2$.

In one embodiment, the polyether polymers of the present invention comprise at least 95% of monomer units that have the structure of formula (I), the residues R are selected from hydrogen and $R^1$ and 50 to 90 % of the residues R are hydrogen and 10 to 50% of the residues R are $R^1$.

In one embodiment, all monomer units of the polyether polymers of the present invention have the structure of formula (I), the residues R are selected from hydrogen and $R^1$ and 60 to 82 % of the residues R are hydrogen and 18 to 40% of the residues R are $R^1$.

A further aspect of the polymers of the present invention is that crystallinity, i.e. degree of crystallization, in comparison to PEG is reduced while retaining the same properties. Some of the polymers have amorphous structure depending on the specific composition. Statistical copolymers of EO with GME or other glycidyl ethers can systematically reduce or completely disable crystallization of PEG. The degree of crystallization of PEG and the related melting point (Table 5) plays a key role in numerous pharmaceutical applications, such as e.g., suppositories or solid dispersions of drugs. In other applications, crystallization is undesired, and fully amorphous PEG is required, for instance in highly hydrophilic and biocompatible polyurethane soft foams used among other purposes for wound dressing. Due to the random incorporation of side chains, the copolymers of this invention permit to obtain fully amorphous PEG copolymer structures. This feature is relevant for biomaterials based on triblock and multiblock structures, for instance combining the polyether with biodegradable polylactide blocks.

Due to their high spatial requirement, the rests $R^1$ provide have a strong effect on crystallinity and polymers with

high content of residues $R^1$ provide amorphous polymers that are otherwise similar to polyethylene glycol. Preferred are therefore polyether polymers of the present invention, wherein 10 to 50 % of residues R are residues $R^1$. Even more preferred are polyether polymers of the present invention, wherein 18 to 40 % of residues R are residues $R^1$. Especially preferred are polyether polymers of the present invention, wherein 10 to 50% of the residues R are $R^1$ and the remaining residues R are hydrogen and most preferred are polyether polymer of the present invention, wherein 18 to 40% of the residues R are $R^1$ and the remaining residues R are hydrogen. These copolymers combine most of the aforementioned advantageous properties. They have low immunogenicity, good water solubility, and low crystallinity and are otherwise very similar to PEG.

n is in the range of 10 to 1000; n is preferably in the range of 20 to 900 and more preferably in the range of 30 to 800. Most preferably n is in the range of 30 to 700. These ranges correspond to the polymer weights of PEG that are most suited for medical applications and that are used in existing medical application for PEG. Furthermore, the polymers of the present invention have preferably a molecular weight $M_n$ as determined by MALDI-TOF MS in the range of 1,000 to 50,000 g/mol, more preferably in the range of 2,000 to 50,000 g/mol, even more preferably in the range of 2,000 to 30,000 g/mol and most preferably in the range of 2,000 to 9,000 g/mol. These ranges apply to all embodiments of the present invention.

The polyether copolymers of the present invention may be random copolymers or statistical copolymers. Such copolymers provide the lowest immunogenicity, as they do not provide a blueprint for the immune system for antibodies. They are intrinsically resistant to an immune response and are therefore preferred embodiments of the present invention. These polymers may be prepared by a process of the present invention as discussed below.

As described above, similarity to PEG is an important feature of the polymers of the present invention. This is especially true for the end groups of the polymer. Processing of PEG for the use in the pharmaceutical field, involves regularly the modification of the end groups. For this purpose, any end group known to be used for PEG can also be used for the present polymers. This includes not only the use of all known end groups for PEG, but also their modification including the kind of modification and the process of modification. If necessary, in order to allow the present polymers to function in this regard as similar or in fact identical to PEG, the present polymers may be provided with ethylene oxide repeating units on either end or both ends of the polymer, as depicted in Formula (Ia) to (Ic). These are preferred embodiments of the present invention.

$$-(CH_2CH_2\text{-}O\text{-})_e\text{-}(CH_2CHR\text{-}O\text{-})_n\text{-} \quad (Ia)$$

$$-(CH_2CHR\text{-}O\text{-})_n\text{-}(CH_2CH_2\text{-}O\text{-})_e\text{-} \quad (Ib)$$

$$-(CH_2CH_2\text{-}O\text{-})_e\text{-}(CH_2CHR\text{-}O\text{-})_n\text{-}(CH_2CH_2\text{-}O\text{-})_e\text{-} \quad (Ic)$$

In these formulae the partial formula $-(CH_2CHR\text{-}O\text{-})_n\text{-}$ depicts a polymer of the present invention as described herein, n is preferably 10 to 900 and e may be any number, but is preferable a number in the range of 1 to 10 and more preferably in the range of 1 to 3. In any case the indices n and e must be chosen such that the molecular weight requirements as stated herein are met, i.e. the number average molecular weight of Polymers (Ia) to (Ic) must be in the range of 1,000 to 50,000 g/mol as determined by MALDI-TOF MS. Polymers according to formulae (Ia) to (Ic) allow the modification of the termini of the present polymers exactly as PEG is modified in known processes and especially in present industrial processes. This allows for a smooth transition of established processes from PEG to the polymers of the present invention. Such can be prepared without much additional effort, as will be discussed below. The preferred molecular weight of the polyether copolymers of formulae (Ia), (Ib) and (Ic) and of the polyether copolymers of formulae (II), (IIa), (IIb) and (IIc) is the same as for polyether copolymers of formula (I), including the preferred ranges.

The polymers of the present invention have dispersities of 1.15 or less. The dispersities of the present invention were determined using size exclusion chromatography (SEC) in DMF by calibration with PEG standards as described in more detail in the examples. They are suitable for applications where low dispersities are required, as for example in pharmaceutical, medical and biological applications. Dispersity of polymers in pharmaceutical, medical and biological applications is of great importance, as macromolecules of different molecular weight may behave differently in biological systems. They may for example differ in solubility in water, membrane permeability, crystallinity and renal clearance. Since a uniform behavior of all molecules is desired in medical applications, a low dispersity is often required. Preferred are therefore polymers of the present invention that have a dispersity of 1.15 or less. Most preferred are polymers of the present invention that have a dispersity of 1.10 or less. This applies to all embodiments of the present invention. A dispersity of 1.10 or less is required for most medical and pharmaceutical applications. Most preferred are polymers of the present invention which have a dispersity that is lower than 1.08.

In another embodiment block copolymers of polyether polymers of the present invention and poly(L-lactic acid) (PLLA) are preferred having -O-CH3, -O-Bz, or -OH as functional group at the end of the polymer chain, i.e. diblock polymers MeO-A-b-PLLA-OH, BzO-A-b-PLLA-OH or HO-A-b-PLLA-OH and HO-A-b-PLLA-OH and triblock polymers such as HO-PLLA-b-A-b-PLLA-OH, wherein A is a polyether polymers of the present invention.

In all embodiments of the invention n is preferably in the range of from 10 to 100 for formula (I) and 9 to 999 for formulae (Ia), (Ib), (Ic) and (II), more preferably in the range of 30 to 800, as long as the aforementioned requirements for the molecular weight of the inventive polymers are met. Most preferably n is in the range of 30 to 700 for all polymers of formulae (I), (Ia), (Ib), (Ic) and (II). These ranges correspond to the polymer weights of PEG that are most suited for medical applications and that are used in existing medical application for PEG. The molecular weight is as stated above, including the preferred ranges.

The dispersity of all compounds of the present invention is 1.15 or less measured by size exclusion chromatography in DMF by calibration with PEG standards and preferably 1.10 or less. Preferably the end group fidelity of the polymer in regard to group X and group Y is at least 95% and the dispersity is 1.10 or less and most preferred wherein the end-group fidelity of the polymer in regard to group X and group Y is at least 98% and the dispersity is 1.10 or less.

As aforementioned, the polymers of the present invention have similar properties as PEG, especially in biological systems. The present polymers may therefore substitute PEG in most of his applications. The polymers of the present invention are especially designed for use in the pharmaceutical and medical field. They may also be used with the same benefits in veterinary medicine and in other biological applications. In these fields the polymers of the present invention can fulfill the same functions as PEG but have reduced immunogenicity and antigenicity. Because of their irregular structure, they do not elicit a strong immune response. The inventive polymers show especially a low response to anti-PEG antibodies, as evidenced by ELISA tests. Conjugates used for masking drugs and other pharmaceutically active compounds usually comprise PEG in addition to a physiologically active compound or an adjuvant. The polymers of the present invention may be used instead of PEG in any such application. At the same time the immune response elicited by the present polymers will be weak or non-existent thereby resolving one of the main problems in the use of PEG.

A further embodiment of the present invention is therefore a conjugate comprising a polyether polymer of any of the preceding claims and a substrate. The conjugates of the present invention may in the following also be named polyether-conjugated substrates or polymer-conjugated substrates. In the conjugates of the present invention a polymer of the present invention is bonded to a substrate directly or indirectly by a covalent bond. The polymer of the present invention may be bonded to the substrate by a spacer, which constitutes an indirect bond. The substrate may be selected from the group consisting of a pharmaceutically active compound, an adjuvant, a surface and interfaces. The pharmaceutically active compound is preferably selected from the group consisting of low molecular weight drugs, peptides, polypeptides, proteins, glycoproteins, polynucleotides and polysaccharides. The adjuvant is preferably a vesicle or carrier, which are preferably used to carry a pharmaceutically active compound. The adjuvant is preferably selected from the group consisting of nanocarriers, liposomal structures, lipid structures and liposomes. Nanocarriers usable in the present invention are for example selected from the group consisting of carbon nanotubes (CNT), dendrimers, gold nanorods, lipid nanoparticles, liposomes, micelles, nanocrystals, niosomes, polymeric nanoparticles (PNP), solid lipid nanoparticles (SLNs) and virus-based nanoparticles (VNP). In a further preferred embodiment the substrate is selected from the group consisting of low molecular weight drugs, nanocarriers, liposomal structures, peptides, polypeptides, proteins, glycoproteins, polynucleotides, polysaccharides, lipid structures, liposomes, surfaces and interfaces. Also in these embodiments the substrate may be bonded directly by a covalent bond or by a spacer to the polymer. Especially preferred are conjugates of the present invention with bovine serum albumin.

In the conjugates of the present invention the polyether polymer comprises monomer units that have a structure of formula (I):

$$-[CH_2\text{-}CHR\text{-}O]_n- \qquad (I),$$

wherein residues R are selected independently from each other from the group consisting of hydrogen, $R^1$ and $R^2$; wherein residues $R^1$ are selected independently of each other from the group consisting of

$$-CH_2\text{-}O\text{-}(CH_2\text{-}CH_2\text{-}O)_m\text{-}R^3,$$

$$-CH_2\text{-}O\text{-}(CH_2\text{-}CH_2\text{-}O)_x\text{-}CH(CH_2\text{-}O\text{-}(CH_2\text{-}CH_2\text{-}O)a\text{-}R^3)(CH_2\text{-}O\text{-}(CH_2\text{-}CH_2\text{-}O)_b\text{-}R^3),$$

$$-CH_2\text{-}O\text{-}(CH_2\text{-}CH_2\text{-}O)_y\text{-}CH_2\text{-}CH(O\text{-}(CH_2\text{-}CH_2\text{-}O)_c\text{-}R^3)\text{-}CH_2\text{-}O\text{-}(CH_2\text{-}CH_2\text{-}O)_d\text{-}R^3,$$

wherein m are independently of each other 1, 2, 3, 4 or 5 and a, b, c, d, x and y, are independently from each other 0 or 1; wherein $R^2$ is $-CH_2\text{-}O\text{-}R^3$,
wherein residues $R^3$ are selected independently of each other from the group consisting of hydrogen, methyl, ethyl, n-propyl and iso-propyl;
and wherein at least 3 % of the residues R are residues $R^1$, based on the total number of all residues R in the polymer. The dispersity of the polyether polymers is preferably 1.15 or less as measured by size exclusion chromatography in DMF by calibration with PEG standards. The number average molecular weight is preferably in the range of 400 g/mol

to 50,000 g/mol. Most preferably the polymers have a dispersity of 1.15 or less and a number average molecular weight in the range of 400 g/mol to 50,000 g/mol. Furthermore, the conjugates of the present invention comprise preferable the polyether polymers of the present invention as described herein, including the preferred embodiments.

The polymer of the present invention may be bonded to the substrate directly by a covalent bond or by a spacer or any kind. Preferably a hydroxy-residue at the ω-terminal of the polymer of the present invention is used to bond a substrate or a spacer to the polymer. This is most economical, as the polymers of the present invention where X is an inert group and Y is OH can be most easily be prepared (see Examples). The conjugation of a substrate to the OH group at the ω-terminal end of PEG is well established. The same processes may be used for the polymers of the present invention. The substrate may however also be bonded to the $\alpha$-terminal of the polymer. The polymers of the present invention can also be bonded to more than one substrate. This can be done by use of the $\alpha$-terminal and the ω-terminal of the polymer and by using functional end groups of the polymer or spacer that can bond to a plurality of substrates. This may also be achieved by use of comonomers with functional groups to which a substrate may be bonded. The bond between the polymers of the present invention and the substrate may be prepared by any means currently used for bonding PEG to substrates. It is also possible to bond a plurality of macromolecules of the polymers of the present invention to one substrate. This is especially useful for vesicle of all kinds including liposomes.

Typical examples for PEG conjugates are the lipid nanoparticles that are essential for vaccines against Covid-19 of the firms BioNTech SE and Pfizer Inc. and Moderna. It comprises a PEG conjugate of lipids, and the lipid nanoparticles (LNPs) are used to encapsulate and protect mRNA, which is the pharmaceutically active substance. The PEG in these conjugates may be substituted by the polymers of the present invention, thereby retaining the effects of the PEG polymer in the conjugate and at the same time reducing the immune response to the conjugate. A further vaccine of this kind is the Covid-19 vaccine of Curevac. Further drugs that comprise PEG conjugates wherein the PEG could be substituted by the present polymers are listed in the following.

Table 1

| Trade names | Chemical Structure | Indication |
|---|---|---|
| Jivi® | 60k-PEG recombinant Factor VII antihemophilic factor | Hemophilia A |
| Palynziq® | 2K-PEG-rhu-Phenylalanine ammonia-lyase, Pegvaliase-pqpz | Phenyl-ketonuria |
| Adynovate® | 20K-PEG-Factor VIII Antihemophilic Factor VIII | Hemophilia A |
| Revolixys® kit | 40K-PEG-RNA aptamer + reverse agent, Factor-IXa blocker, Pegnivacogin /Anivamersen | Anti-coagulation |
| Onicyde® | 2K-PEG-Liposomal irinotecan hydrochloride trihydrate | Metastatic pancreatic cancer |
| Plegridy® | 20k PEG-Interferon β-1a | Relapsing from multiple sclerosis |
| Movantic® | <1k PEG-Naloxegol | Opioid-induced constipation |
| Omontys® | 40K-PEG-Erythropoietin-mimetic peptide, Peginesatide | Anemia associated with chronic kidney disease |
| Sylatron® | 12K-PEG-Interferon a-2b | Melanoma |
| Krystexxa® | 10k-PEG-Uricase, Pegloticase | Gout |
| Cimzia® | 40k-PEG-Certolizumab | Rheumatoid arthritis, Crohn's disease, Axial spondyloarthritis and psoriatic arthritis |

(continued)

| Trade names | Chemical Structure | Indication |
|---|---|---|
| Mircera® | 30K-PEG- erythropoietin (epoetin) β | Anemia associated with chronic kidney disease |
| Macugen® | 40K-PEG-anti-VEGF aptamer, Pegaptanib | Age-related macular degeneration |
| Somavert® | 5K-PEG-rhuGH (human growth hormone), Pegvisomant | Acromegaly |
| Neulasta® | 20K-PEG-Granulocyte colony stimulating factor, Pegfilgrastim | Neutropenia |
| Pegasys® | 40K-PEG-interferon α-2 | Hepatitis C und B |
| PegIntron® | 12K-PEG-interferon a-2b | Hepatitis C und B |
| Doxil®/Caelyx® | 2K-PEG-Liposomal doxorubicin HCl | Cancer |
| Oncaspar® | 5K-PEGylated L-asparaginase, Pegaspargase | Acute lymphoblastic leukemia |
| Adagen® | 5K-PEG-adenosine deaminase (bovine), Pegademase | Severe combined immunodeficiency disease (SCID) |
| Irinotecan® | PEGylated liposomal irinotecan hydrochloride trihydrate | Metastatic pancreatic cancer |
| Lonquez® | 20k PEG-rhG-CSF | Neutropenia |
| Jintrolong®[1] | Branched 40k PEG hGH | hGH deficiency |
| Neulapeg® | 20k-PEG rhG-CSF mutein | Neutropenia |
| Rebinyn®/ Refixia® | Branched 40k PEG Recombinant factor VIII | Hemophilia B |
| Asparlas® | 5k-PEG L-Asparaginase | Acture lymphoblastic leukemia |
| Revcovi® | 5k-PEG Recombinant B-domain | Hemophilia A |
| | truncated factor VIII | |
| Besremi®[3] | Branched 40k PEG Proline-interferon a-2b | Polycythemia vera |
| PEGPH20®[3] | 30k-PEG recombinant human hyaluronidase | Pancreatic cancer |
| Pegargiminase | 20k PEG Arginine deiminase Interleukin-2 | Hepatocellular carcinoma |
| NKTR-214 | Branched PEG-20k Fmoc-succinimidyl carbonate | Melanoma, rencal cell carcinoma |
| PRX-201[3] | Bifunctional PEG2k α-Galactosidase A | Fabry disease |
| Peginterferon lambda-1a | 20k PEG recombinant interferon lambda-1a | Hepatitis C and B[2], Hepatitis D[3] |
| Abicipar pegol | 20k PEG anti-VEGF DARPin | Neovascular age related macular degeneration |
| TransCon GH®[3] | Four-arm-40PEG spacer hGH | Growth hormone deficiency |

(continued)

| Trade names | Chemical Structure | Indication |
|---|---|---|
| AM0010[2] | 30k PEG-Interleukin-10 | Metastatic pancreatic cancer |
| Rolontis®[3] | Bifunctional PEG rhG-CSF | Neutropenia |
| Efpeglenatide[3] | Bifunctional PEG Exendin-4 (glucagon-like peptide-1 receptor agonist) | Type-2 diabetes mellitus |
| Pegbelfermin[2] | 30k PEG fibroblast growth factor-21 mutein | Fibrosis, liver disorders, nonalcoholic steatohepatitis, type 2 diabetes mellitus |
| Imrestor® (veterinary use) | 20k PEG recombinant bovine G-CSF mutein | Reducing risk of clinical mastitis |
| Pegfilgrastim biosimilars: Fulphila (Mylan®) Lapelga/Pelgraz (Apobiologix®), Udenyca (Coherus Biosciences®), Pelmeg (Mundipharma®), Ziextenzo (Sandoz®) | 20K-PEG-Granulocyte colony stimulating factor, Pegfilgrastim, rhG-CSF | Neutropenia |
| Esperoct® | Branched-PEG40k-SA-CMP, enzymatic glycopegylation at O-glycan on Ser-750 | Hemophilia A |
| [1] only in China<br>[2] Phase II<br>[3] Phase III | | |

In another embodiment of the invention the invention relates to a process for the preparation of a polyether polymer by anionic ring-opening copolymerization (AROP) comprising the steps of:

- Providing an anion $An^-$,
- Adding at least one monomer of the formula

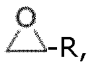

wherein R is as defined for any of formulae (I),

- Allowing the polymerization to proceed at a temperature in the range of -10 to 90 °C.

In the process of the present invention the anion $An^-$ must be an anion suitable for anionic ring-opening copolymerization (AROP). The expert in the art can easily choose among the known anions for this purpose. The $An^-$ anion may be selected from the groups consisting of alkyl anion, hydride, $OH^-$, alkoxy, $SH^-$, thioalkoxy$^-$, amine anion, amide anion, imide anion and combinations thereof. Alkyl anions and hydride anions may be provided in the form of metal alkyl or metal hydride compounds. The alkoxy anion and the thioalkoxy anion may be the corresponding anion with the same structure as any of the alkoxy groups and aryloxy groups and aralkoxy groups defined herein for the residue X. Preferably however the alkoxy anion and the thioalkoxy anion are not tertiary alkoxy anions. The imide anion is preferably a phthalimide anion. Polymers of the Formulae (Ia), (Ic), (IIa) and (IIc) may be prepared by first adding the corresponding amount of ethylene oxide to the anion $An^-$ and allowing the polymerization to proceed at a temperature in the range of -10 to 90 °C until all of the ethylene oxide is consumed. Subsequently the steps of adding least one monomer of the formula

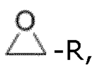

and allowing the polymerization to proceed at a temperature in the range of -10 to 90 °C are performed. In this manner the

repeating units adjacent to the $\alpha$ -terminal of the polymers of the present invention are provided with exactly the same structure as PEG. Polymers of the Formulae (Ia) and (Ic) may also be prepared by use of anions of the formula $X-(CH_2CH_2-O-)_a^-$, wherein a is as defined for any of the Formulae (Ia) and (Ic) and X is any residue as defined herein for X. X' is any residue as defined herein for X'. This is the preferred method. Especially preferred is the use of such anions of the formula $X-(CH_2CH_2-O-)_a^-$, in which X is alkyl, alkoxy, dialkylamin anion or $(RCO)_2N^-$. Even more preferred is the use of anions selected from the group consisting of $MeOCH_2CH_2O^-$, $MeO(CH_2CH_2O)_2^-$, $BenzylOCH_2CH_2O^-$, $BzO(CH_2CH_2O)_2^-$, $(Bz)_2N-CH_2CH_2O^-$, $(Bz)_2N-(CH_2CH_2O)_2^-$, phthalimide-$CH_2CH_2O^-$, phthalimide-$(CH_2CH_2O)_2^-$, (1,3-dimethoxypropan-2-yl)oxy anion, (2,3-dimethoxypropan-1-yl)oxy anion, 2-((1,3-dimethoxypropan-2-yl)oxy)ethan-1-oxy anion, (2,5,8-trioxanonan-3-yl)methyloxy and (2,6,9-trioxadecan-4-yl)methyloxy anion. (2,5,8,11-tetraoxadodecan-6-yl) methyloxy anion and (2,5,9,12-tetraoxatridecan-7-yl)oxy anion, wherein Me is methyl and Bz is benzyl. Most preferred are $MeO(CH_2CH_2O)_2^-$, $BzOCH_2CH_2O^-$ and $(Bz)_2N-CH_2CH_2O^-$, (1,3-dimethoxypropan-2-yl)oxy anion, (2,3-dimethoxypropan-1-yl)oxy anion, 2-((1,3-dimethoxypropan-2-yl)oxy)ethan-1-oxy anion, (2,5,8-trioxanonan-3-yl)methyloxy and (2,6,9-trioxadecan-4-yl)methyloxy anion. (2,5,8,11-tetraoxadodecan-6-yl)methyloxy anion and (2,5,9,12-tetraoxatridecan-7-yl) oxy anion.

In addition, in order to synthesize bifunctional initiated copolymers that enable the synthesis of ABA triblock copolymers, a polyether polymer of the present invention bearing two hydroxyl-termini is used as an macroinitiator for ring-opening polymerization of cyclic polyesters, cyclic diesters or cyclic monoesters, e.g. L-lactide, D-lactide, meso-lactide, cyclic carbonates, such as trimethylene carbonate (TMC), 2,2-dimethyl-trimethylene carbonate (DTC), 5-butyl-1,3-dioxan-2-one and 1,3-dioxepan-2-one; glycolide, other cyclic esters and lactams, *N*-carboxy anhydrides. In an embodiment of the invention the reaction is performed using L-lactide enabling the synthesis of e.g. PLLA-*b*-P(EG-*co*-GME)-*b*-PLLA, wherein PLLA means

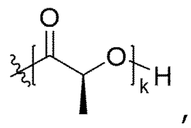

wherein k = 5-500, preferably 5-250, most preferably 10-200.

The polymerization is catalyzed by Lewis acids e.g. triethylaluminum ($AlEt_3$) and tin(II) bis-(2-ethylhexanoate) (Sn(Oct)2) or organic bases e.g., DBU (1,8-Diazabicyclo[5.4.0]undec-7-ene) and *N*-methyl-1,5,7-triazabicyclo[4.4.0] dec-5-ene. The described procedure is not limited to triblock copolymers. With the utilization of a monofunctional P(EG-*co*-GME) macroinitiator, diblock copolymers (P(EG-*co*-GME)-b-PLLA) are obtained.

In these embodiments $An^-$ can be selected from the group consisting of alkoxy$^-$, thioalkoxy$^-$ and amine anion.

Further, in a final step of the preparation, addition of a small amount of pure EO is possible to ensure primary hydroxyl end groups for the $\omega$-terminal of the polymer that can undergo all established coupling strategies to therapeutic entities. This leads to polymers of the present invention with Formulae (Ib) and (Ic).

The counter ion to the $An^-$ anion is preferably selected from the group consisting of $Na^+$, $K^+$ and $Cs^+$. The anion An- may be provided in an inert solvent.

The anionic ring-opening polymerization of epoxides can be conducted in non-protic solvents like DMSO or other polar aprotic solvents, e.g., tetrahydrofuran (THF), methyl-THF, cyclopentyl methyl ether (CPME), benzene, toluene, xylene, HMPA, dioxane, diglyme etc. Surprisingly, it was found that random copolymers can be synthesized by using the non-protic solvent DMSO and statistical copolymers can be synthesized by using polar aprotic solvents e.g. toluene. This can be seen from figures 9, 10, 14 and 15.

The solvent is preferably a non-protic solvent and most preferably dimethyl sulfoxide (DMSO). Also preferably the reaction is performed in the same solvent.

Anionic ring-opening polymerizations with alkyl glycidyl ethers are more prone to undergo chain transfer reactions as follows:

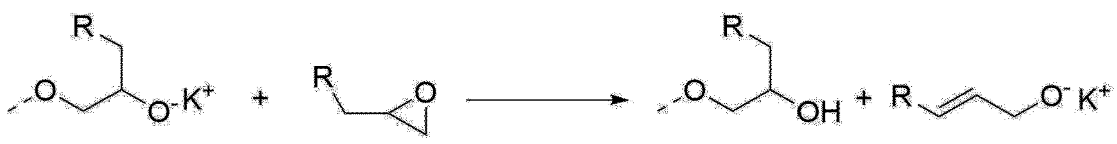

These side reactions increase with the temperature. Therefore, high molecular weight polymers can only be obtained, if the reaction temperature is not above 90°C. The chain transfer reaction results in a broad distribution of molecular weight and therefore in a high dispersity. Therefore, lower polymerization temperatures also provide polymers of the present invention with lower dispersity. On the other hand, alkyl glycidyl ethers are as reactive in the anionic ring-opening polymerization as ethylene oxide. For this reason the polymerization may be performed at temperatures as low as -10°C. Preferably the polymerization is performed at a temperature in the range of 10 to 70°C and more preferred at a temperature in the range of 10 to 60°C.

A further problem in anionic ring-opening polymerizations is that commercially available alkyl glycidyl ethers are prepared from epichlorohydrin. Part of the epichlorohydrin remains in the alkyl glycidyl ether. Presence of alkyl glycidyl ethers leads to termination reactions as follows:

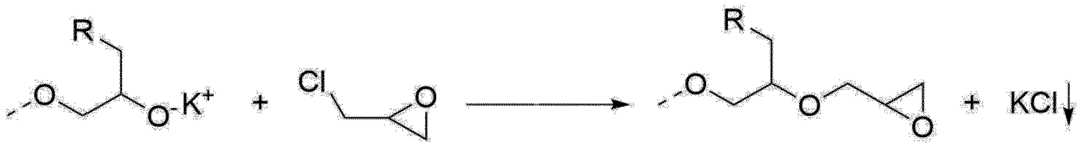

This in turn leads to low molecular weights and high dispersity of the product. Some of the polymers of the present invention, especially those, where R is $R^2$ are therefore only accessible by reaction temperatures of -10 to 90°C or less and by the use of alkyl glycidyl ethers that comprise less than 1 wt% of epichlorohydrin. Preferably the alkyl glycidyl ethers comprise less than 0.5 wt% of epichlorohydrin and more preferably less than 0.1 wt%. Most preferably the alkyl glycidyl ether are free of epichlorohydrin. Removal of epichlorohydrin from the glycidyl methyl ether to the extent necessary is impossible, because the glycidyl methyl ether and epichlorohydrin have similar boiling points, e.g. epichlorohydrin 117.9°C, glycidyl methyl ether 110°C. A procedure for the preparation of epichlorohydrin free alkyl glycidyl ethers is provided in Example 1 of WO 2022/238532 A1. The method may be applied to all monomers used for the preparation of the polyether polymers of the present invention.

Furthermore, high temperatures also lead to side reactions of the starting anion $An^-$ or the group X of the living polymer. Limitation of the temperature in the aforementioned range therefore also improves the end-group fidelity at the $\alpha$-terminal of the polymers of the present invention and allows for end-group fidelities of 95% or more of the X residue. Other known methods for the polymerization of the alkyl glycidyl ethers do not provide the low dispersity required for the present polymers. They also do not allow for the same high end-group fidelity.

Hydroxyl groups in the side chains may be introduced into the polymers of the present invention by use of monomers that have a hydroxyl group that is protected by a protective group, such as for example methoxymethyl ether, tetrahydropyranyl ether, vinyl ether or any other suitable protective group known in the art. After preparation of the polymers, the protective group may be removed to provide a polyether polymer of the present invention with a hydroxyl group in a side chain.

In order to provide random or statistical copolymers or the present invention, it is necessary to add at least two different kinds of monomers to the anion $An^-$. The reactivity ratio for both alkyl glycidyl ether employed and ethylene oxide is in the range of 0.7 to 1.3 or 0.6 to 1.7.

A further embodiment of the present invention is the use of the polyether polymers as described for the conjugates of the present invention for the preparation of a conjugate of the polymers with a substrate. The substrate is as defined herein for the conjugates of the present invention. The substrate is preferably a bioactive compound. There is a well-developed chemistry for the preparation of conjugates of PEG, which is known to the expert in the art. The same chemistry may be used for the preparation of a conjugate of the present invention. Preferred is a use of the present invention, for the preparation of conjugated lipids for use in vaccines, in particular based on lipid nanoparticles. These nanoparticles are preferably nanoparticles, as used against COVID-19. Preferred are conjugates of polymers of the present invention with bovine serum albumin (BSA). Especially preferred are conjugates with polymers of the present invention where the y group of the polymer is a N-succinimidyl carbonate group. Even more preferred are conjugates of the present polymer where bovine serum albumin in conjugates by means of such a group Y to a polymer of the present invention.

An additional embodiment for the present invention concerns the preparation of conjugates of the present invention. As discussed above, the conjugates may be prepared by any known process for the preparation of conjugates of PEG. The present invention provides additionally a process, where firstly the process for the preparation of the polymers of the present invention is performed and then a substrate is added to the living polymer, wherein the substrate has a functional group that reacts with the living polymer to form a conjugate.

Abbreviations:

| | |
|---|---|
| EG | ethylene glycol |
| DCM | dichloromethane |

| | |
|---|---|
| DMP2GE | 2-(((1,3-dimethoxypropan-2-yl)oxy)methyl)oxirane |
| DMPEOH | 2-((1,3-dimethoxypropan-2-yl)oxy)ethan-1-ol |
| DMSO | dimethyl sulfoxide |
| EO | ethylene oxide |
| GE | glycidyl ether |
| GME | glycidyl methyl ether |
| scEO | ethylene oxide monomers with side chains |
| scPEG | poly(ethylene glycol) polymers with side chains (polymers of the present invention) |
| SEC | size-exclusion chromatography |
| MALDI ToF MS | matrix-assisted laser desorption/ionization time-of-flight mass spectrometry |
| ELISA | enzyme-linked immunosorbent assay |

Brief description of the figures:

| | |
|---|---|
| Figure 1: | Figure 1 shows stacked SEC traces of polymers of the present invention synthesized in DMSO. |
| Figure 2: | Figure 2 shows the SEC trace of a polymer of the present invention synthesized in DMSO. |
| Figure 3: | Figure 3 shows the MALDI-TOF mass spectrum of a polymer of the present invention synthesized in DMSO. |
| Figure 4: | Figure 4 shows the $^1$H NMR spectrum of a polymer of the present invention synthesized in DMSO. |
| Figure 5: | Figure 5 shows turbidity measurements of two polymers of the present invention synthesized in DMSO. |
| Figure 6: | Figure 6 shows stacked $^1$H NMR spectra of copolymerization of EO and DMP2GE in DMSO-$d_6$ at different times of the copolymerization. |
| Figure 7: | Figure 7 shows the result of a study of the relative reactivities of ethylene oxide and DMP2GE in the anionic ring opening polymerization. |
| Figure 8: | Figure 8 shows the enzyme-linked immunosorbent assays of mPEG 5k, P(EG-*co*-GME) and scPEGs. |
| Figure 9: | Figure 9 shows the SEC trace of a polymer of the present invention synthesized in DMSO. |
| Figure 10: | Figure 10 shows the MALDI-TOF mass spectrum of a polymer of the present invention synthesized in DMSO. |

**Examples:**

Reagents:

Chemicals were purchased from TCI, BLDpharm, Acros Organics, Roth, Sigma Aldrich and Honeywell, unless otherwise noted. Ethylene oxide was obtained from Air Liquide. Glycidyl methyl ether was prepared according to WO 2022/238532 A1, Example 1b. Deuterated solvents were purchased from Deutero GmbH. THF was flashed over basic aluminum oxide before usage. Monomers were either dried over $CaH_2$ and cryo-transferred or dried over benzene before the polymerizations.

Measurements:

$^1$H and $^{13}$C NMR spectra were recorded on a Bruker Avance III HD 300 or 400 spectrometer with 300 or 400 MHz, respectively, and referenced internally to residual proton signals of the deuterated solvent.

$M_w$, $M_n$ and dispersities ($M_w/M_n$ = PDI) of all samples were determined from the corresponding size exclusion chromatograms (refractive index (RI) detector, DMF, calibration with PEG standards).

Size-exclusion chromatography (SEC) measurements were performed with dimethylformamide (DMF with 1 g/L LiBr) as the mobile phase (flow rate 1 mL/min) on poly(2-hydroxyethylmethacrylat) (PHEMA) 300/100/40 columns at 50 °C. Polymer concentrations were 1 mg/mL. Calibration was carried out using poly(ethylene glycol) standards (from Polymer Standard Service, Mainz, Germany).

Differential scanning calorimetry (DSC) measurements were carried out in the temperature range of -90 to 70 °C with a heating rate of 10 K/min at a TA Instruments DSC 250. The thermal history of the samples was excluded via two cooling and two heating cycles. For each sample, the glass transition and the melting temperatures were obtained from the second heating curve.

MALDI-ToF MS measurements were carried out at a Bruker autoflex maX MALDI-TOF/TOF. The potassium salt of trifluoroacetic acid and trans-2-[3-(4-tert-Butylphenyl)-2-methyl-2-propenylidene]malononitrile (DCTB) were utilized as ionization salt and matrix, respectively.

Example 1: Synthesis of 2-(((1,3-dimethoxypropan-2-yl)oxy)methyl)oxirane (DMP2GE)

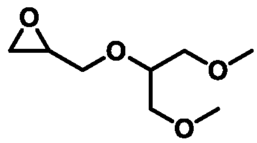

A round bottomed flask equipped with a magnetic stirrer was charged with epichlorohydrin (31.3 g, 26.5 mL, 336 mmol) and cooled with an ice bath. Finely grounded NaOH (20.2 g, 504 mmol) was added under stirring. 1,3-Dimethoxypropan-2-ol (20.2 g, 20.0 mL, 168 mmol) was added and the solution was heated to 30 °C. After 5 h, diethyl ether (100 mL) was added. The reaction mixture was filtered and the filtrate was dried over $MgSO_4$. After an additional filtration step, solvent and excess epichlorohydrin was evaporated under high vacuo. 2-(((1,3-dimethoxypropan-2-yl)oxy)methyl)oxirane (13.4 g, 76.2 mmol, 45%) was obtained after fractional distillation as a colorless liquid.

Example 2: Synthesis of 2,5,9,12-tetraoxatridecan-7-ol

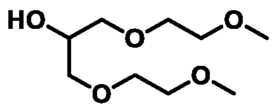

Epichlorohydrin (14.2 g, 12.0 mL, 153 mmol) and 2-methoxy ethanol (46.6 g, 48.0 mL, 612 mmol) were mixed in a three-necked flask equipped with a magnetic stirrer, dropping funnel and reflux condenser. The solution was heated to 50 °C and 50 wt% NaOH solution (8 mL) was added dropwise under stirring. The reaction mixture was heated to 80 °C and stirred overnight. After cooling to room temperature. The reaction mixture was filtered. The filtrate was neutralized with concentrated HCl and extracted with dichloromethane (DCM, 25 mL, five times). The combined organic phases were dried with $MgSO_4$. After filtration, the solvent was evaporated under vacuo. 2,5,9,12-tetraoxatridecan-7-ol (7.40 g, 35.5 mmol, 23%) was obtained as a colorless liquid after fraction distillation.

Example 3: Synthesis of 2-(((2,5,9,12-tetraoxatridecan-7-yl)oxy)methyl)oxirane (DMEP2GE)

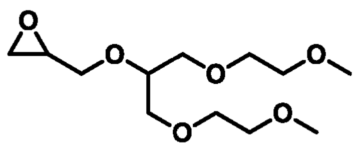

A round bottomed flask equipped with a magnetic stirrer was charged with epichlorohydrin (6.61 g, 5.60 mL, 71.4 mmol) and cooled with an ice bath. Finely grounded NaOH (4.23 g, 106 mmol) was added under stirring. 2,5,9,12-tetraoxatridecan-7-ol (7.40 g, 35.5 mmol) was added and the solution was heated to 30 °C. After 5 h, the solution was cooled to room temperature and diethyl ether (40 mL) was added. The reaction mixture was filtered and the filtrate was dried over $MgSO_4$. After an additional filtration step, solvent and excess epichlorohydrin was evaporated under high vacuo. 2-(((2,5,9,12-tetraoxatridecan-7-yl)oxy)methyl)oxirane (8.67 g, 32.8 mmol, 92%) was obtained after fractional distillation as a colorless liquid.

Example 4: Synthesis of 2-((1,3-dimethoxypropan-2-yl)oxy)ethan-1-ol (DMPEOH)

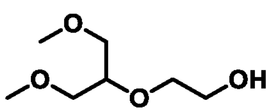

A three necked flask equipped with a reflux condenser and septum was flame dried under high vacuo. NaH (719 mg, 30.0 mmol) was added under argon. The flask was cooled with an ice bath and dry THF (25 mL) was added under argon. The suspension was stirred for 10 min and 1,3-dimethoxypropan-2-ol (3.00 g, 2.97 mL, 25 mmol) was slowly added. After 30 min, 1,3,2-dioxathiolane 2,2-dioxide (3.10 g, 25 mmol) was added in small portions under argon. The resulting reaction mixture was stirred under reflux overnight. After cooling to room temperature THF was evaporated under vacuo. Diethyl ether (25 mL) was added to the residue and diluted $H_2SO_4$ (50 mL) was added under ice cooling and vigorous stirring. After reaching room temperature overnight, the solution was extracted with diethyl ether (25 mL, three times) and the combined organic phases were dried with $MgSO_4$. After filtration, the solvent was evaporated. 2-((1,3-dimethoxypropan-2-yl)oxy)ethan-1-ol (711 mg, 4.33 mmol, 17%) was obtained as a colorless liquid after fractional distillation.

Example 5: General procedure for the preparation of random copolymers with branched side chains

Synthesis of $\alpha$-DMPEO-P(EG$_{0.70}$-*co*-DMP2GE$_{0.30}$) (entry c)

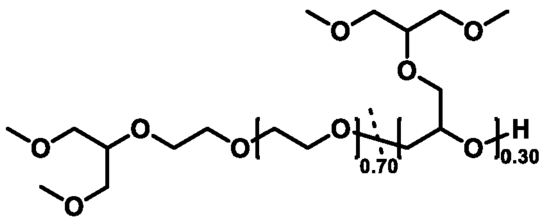

2-((1,3-Dimethoxypropan-2-yl)oxy)ethan-1-ol (59.0 mg, 359 μmol) was dissolved in benzene (5 mL) and transferred via syringe into a flame-dried flask under argon. Potassium-*tert*-butoxide (39.5 mg, 352 μmol) was dissolved in stabilizer-free THF (3 mL) and small quantities of Millipore water and transferred into the flask. High vacuum was applied to the flask and the solvents were removed under high vacuum. The resulting initiator salt was dried under high vacuum at 55 °C overnight and finally dissolved in dry DMSO (5 mL) under stationary vacuum. After freezing the resulting solution at -78 °C, DMP2GE (840 mg, 4.74 mmol) was added via syringe to the flask. EO (488 mg, 503 mL, 11.1 mmol) was added to the flask via cryo-transfer before the cooling bath was removed and the reaction mixture was allowed to warm up to room temperature. The reaction was allowed to stir for 24 h at 30 °C. Subsequently, the flask was vented and most DMSO was evaporated under high vacuo. The polymer was redissolved in a toluene and diethyl ether mixture and acetic acid was added. After filtration, solvents and residual acetic acid were evaporated and $\alpha$ -DMPEO-P(EG$_{0.70}$-*co*-DMP2GE$_{0.30}$) (96%) was obtained as a viscous liquid.

The MALDI-ToF MS of $\alpha$-DMPEO-P(EG$_{0.70}$-*co*-DMP2GE$_{0.30}$) reveals an exceptional high end-group fidelity (>99%), as can be seen in Figure 3. The data of the product can be found in Tables 1 and 2, entry a.

Further polymers of the present invention (scPEG) with varying amounts and different glycidyl ether monomers with branched ether side chains (entry a,b and d) were prepared according to the same procedure (entries a, b, and d of Tables 1 to 4).

Example 6: Competitive Enzyme-linked Immunosorbent Assay (ELISA)

The interaction of the copolymer samples entry b and c, P(EG-co-GME) and of commercially available mPEG 5k were evaluated by a competitive PEG ELISA kit using a murine monoclonal, horseradish peroxidase conjugated anti PEG antibody (HRP anti-PEG) (Life diagnostics, West Chester, PA, USA). Samples of concentrations ranging from 0 to 6.37 mg $ml^{-1}$ were prepared in dilution buffer. Additionally, mPEG with a molecular weight of 5000 g $mol^{-1}$ was utilized as internal standard for comparison. 50 μl of each prepared sample was dispensed to a PEG pre-coated 96-well plate and 50 μl of HRP anti-PEG was added to each well. The solutions were incubated for 1 h at 25 °C with a micro-plate shaker and then washed six times with 300 μl of wash buffer per each well. After removal of residual droplets, 100 μl of 3,3',5,5'-tetramethylbiphenyl-4,4'-diamine was added to each well and the solutions were mixed on a micro-plate shaker for 20 min. The reaction was stopped by addition of 100 μl of stop solution and the absorbance at 450 nm was read within 5 minutes.

Analysis of ELISA Data: The determined absorbance values were normalized to visualize the percent of maximal binding. The sample concentrations were transformed to a function of $\log_{10}$. The sigmoidal fits were calculated using the following equation with a representing the lower, b the upper limit, x0 the inflection point and dx the hill slope.

$$y = a + (b-a)/(1 + \exp((x-x0)/dx))$$

Example 7: Kinetics of the Synthesis of P(EG-*co*-DMP2GE)

The synthesis of P(EG-*co*-DMP2GE) was repeated in fully deuterated DMSO for an online in situ $^1$H NMR kinetics measurement. DMP2GE and ethylene oxide were dissolved in fully deuterated DMSO. The consumption of ethylene oxide and DMP2GE over time was measured by $^1$H-NMR spectroscopy. As measure for the ethylene oxide concentration, the signal of the protons of ethylene oxide at about 2.61 ppm was used. For DMP2GE the signals of the protons at the oxirane ring at about 2.55, 2.71 and 3.08 ppm were used. Figure 6 shows the decrease of the selected $^1$H NMR signals of the two monomers over time. Figure 7 shows the plot of monomer consumption $M_{x,t}/M_{x,t=0}$ versus total conversion. As can be seen, ethylene oxide and DMP2GE are consumed at nearly the same rate. This data shows that the copolymers of the present invention are almost ideal random copolymers.

Example 8: Synthesis of 1-methoxy-3-(2-methoxyethoxy)propan-2-ol (MMEPOH)

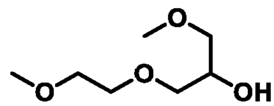

Ethylene glycol monomethyl ether (8.64 g, 9.00 mL, 113 mmol) was added to a flask equipped with a reflux condenser. NaOH solution (19 M, 3 mL) was added under stirring and the resulting solution was heated to 55 °C. Glycidyl methyl ether (5.00 g, 5.10 mL, 56.7 mmol) was added and the solution was stirred overnight. The solution was cooled to room temperature and extracted with dichloromethane (DCM, 50 mL) three times. The combined organic phases were dried over magnesium sulphate ($MgSO_4$). After filtration, the solvent was evaporated under reduced pressure. 1-Methoxy-3-(2-methoxyethoxy)propan-2-ol (3.74 g, 22.7 mmol, 40%) was obtained as a colorless liquid after fractional distillation of the residue.

Example 9: 1-Chloro-3-(2-methoxyethoxy)propan-2-ol

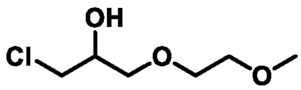

Concentrated sulfuric acid (95%, 2.71 g, 26.3 mmol) was mixed with 2-methoxyethanol (20.0 g, 263 mmol). The mixture was added dropwise to epichlorohydrin (48.6 g, 526 mmol) under stirring while cooling the flask with an ice/water bath. After complete addition of the alcohol, the reaction mixture was stirred at 80°C while the reaction progress was monitored via TLC. After 16 h, water (30 mL) was added to the reaction mixture. The organic layer was separated and the aqueous phase extracted three times with chloroform (20 mL). The combined organic phases were neutralized with saturated sodium hydrogen carbonate solution, dried over sodium sulfate. Chloroform and epichlorohydrin were removed under reduced pressure. The crude product was distilled under high vacuum. 1-Chloro-3-(2-methoxyethoxy)propan-2-ol was obtained as a colorless liquid (11.34 g, 67.3 mmol, 26%).

Example 10: 2-((2-Methoxyethoxy)methyl)oxirane (MEGE)

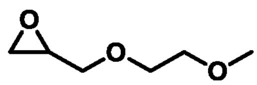

1-Chloro-3-(2-methoxyethoxy)propan-2-ol (11.3 g, 67.3 mmol) was dissolved in diethyl ether (10 mL) and cooled with a water bath. Freshly ground sodium hydroxide (2.96 g, 74.0 mmol) was added to the solution. The reaction progress

was monitored via TLC. After completion, the resulted sodium chloride was filtered and the filtrate was dried over sodium sulfate. After removal of diethyl ether under reduced pressure, the crude product was fractionally distilled under high vacuum yielding 2-((2-methoxyethoxy)methyl)oxirane as a colorless liquid (7.62 g, 57.7 mmol, 86%).

Example 11: General procedure for the preparation of random copolymers with linear side chains

Synthesis of $\alpha$-MMEPO-P(EG$_{0.91}$-*co*-MEGE$_{0.09}$) (entry e)

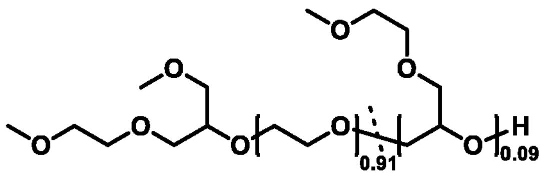

1-Methoxy-3-(2-methoxyethoxy)propan-2-ol (50.0 mg, 300 $\mu$mol) was dissolved in benzene (6 mL) and transferred via syringe into a flame-dried flask under argon. Potassium-tert-butoxide (31.0 mg, 270 $\mu$mol) was dissolved in stabilizer-free THF (3 mL) and small quantities of Millipore water and transferred into the flask. High vacuum was applied to the flask and the solvents were removed under high vacuum. The resulting initiator salt was dried under high vacuum at 60 °C overnight and finally dissolved in dry DMSO (10 mL) under stationary vacuum. After freezing the resulting solution at -78 °C, MEGE (460 mg, 3.47 mmol) was added via syringe to the flask. EO (1.38 g, 1,42 mL, 31.2 mmol) was added to the flask via cryo-transfer before the cooling bath was removed and the reaction mixture was allowed to warm up to room temperature. The reaction was allowed to stir overnight at room temperature. Subsequently, the flask was vented, quenched with acidified MeOH and the solvents were evaporated under high vacuo. The polymer was redissolved in MilliQ water. After lyophilization, $\alpha$-MMEPO-P(EG$_{0.91}$-*co*-MEGE$_{0.09}$) was obtained as a viscous liquid.

Discussion of the results

Tables 1, 2, 3 and 4 and Figure 1 to 5 show the properties of the polymers prepared. They are discussed in the following.

Table 1: Composition of polymers prepared

| *Entry* (Example) | *Sample* | $DP_{EO}$ NMR | $DP_{GE}$ NMR | $mol\%_{EO}$ NMR | $mol\%_{scEO}$ NMR |
|---|---|---|---|---|---|
| a (5) | $\alpha$-DMPEO-P(EG$_{0.92}$-*co*-DMP2GE$_{0.08}$) | 46 | 4 | 92 | 8 |
| b (5) | $\alpha$-DMPEO-P(EG$_{0.80}$-*co*-DMP2GE$_{0.20}$) | 43 | 11 | 80 | 20 |
| c (5) | $\alpha$-DMPEO-P(EG$_{0.70}$-*co*-DMP2GE$_{0.30}$) | 38 | 16 | 70 | 30 |
| d (5) | $\alpha$-DMPEO-P(EG$_{0.83}$-*co*-DMEP2GE$_{0.17}$) | 43 | 9 | 83 | 17 |
| e (11) | $\alpha$-MMEPO-P(EG$_{0.91}$-*co*-MEGE$_{0.09}$) | 84 | 8 | 91 | 9 |

*DP* = degree of polymerization.

Table 2: Characterization data of polymers prepared

| *Entry*/ (Example) | *Sample* | $M_{n,NMR}$ [kg/mol] | $M_{N,MALDI}$ [kg/mol] | $M_{n,SEC}$ [kg/mol] | $PDI_{SEC}$ | *End-group fidelity* MALDI |
|---|---|---|---|---|---|---|
| a (5) | $\alpha$-DMPEO-P(EG$_{0.92}$-*co*-DMP2GE$_{0.08}$) | 2.9 | 2.9 | 2.4 | 1.06 | >99% |
| b (5) | $\alpha$-DMPEO-P(EG$_{0.80}$-*co*-DMP2GE$_{0.20}$) | 4.0 | 4.0 | 3.0 | 1.06 | >99% |
| c (5) | $\alpha$-DMPEO-P(EG$_{0.70}$-*co*-DMP2GE$_{0.30}$) | 4.7 | 4.6 | 3.2 | 1.05 | >99% |
| d (5) | $\alpha$-DMPEO-P(EG$_{0.83}$-*co*-DMEP2GE$_{0.17}$) | 4.4 | 4.4 | 3.3 | 1.10 | >99% |
| e (11) | $\alpha$-MMEPO-P(EG$_{0.88}$-*co*-MEGE$_{0.12}$) | 5.0 | 5.0 | 4.2 | 1.06 | 99% |

$M_n$ = molecular weight (number average molar mass), PDI = dispersity.

Table 3: Thermal properties in aqueous solution

| *Entry*/ (Example) | *Sample* | [scEO] (mol%)[a] | *Cloud Point (°C)* |
|---|---|---|---|
| a (5) | $\alpha$-DMPEO-P(EG$_{0.92}$-*co*-DMP2GE$_{0.08}$) | 8 | N/O* |
| b (5) | $\alpha$-DMPEO-P(EG$_{0.80}$-*co*-DMP2GE$_{0.20}$) | 20 | N/O* |
| c (5) | $\alpha$-DMPEO-P(EG$_{0.70}$-*co*-DMP2GE$_{0.30}$) | 30 | 94 |
| d (5) | $\alpha$-DMPEO-P(EG$_{0.83}$-*co*-DMEP2GE$_{0.17}$) | 17 | N/O* |

*N/O = not observed in the range of 0 to 99 °C.

Table 3 shows the thermal properties of the respective compounds in aqueous solution. The polymers were prepared according to Example 5 with varying amounts of the glycidyl ether monomers with ether side chains (scEO). The cloud point of the random copolymers of ethylene oxide and glycidyl ether monomers with ether side chains were investigated via turbidimetry measurements of copolymers containing different amounts of glycidyl ether monomers with ether side chains. Cloud point was defined at a transmittance of 50 %.

A cloud point of the copolymers was merely observed for 30 mol% DMP2GE, see Figure 5. Despite the decrease in the cloud point with increasing mol% DMP2GE, solubility in water under physiologically conditions is ensured for all copolymers.

Dispersity

As can be seen from Table 2, entries a to d, all polymers of the present invention have a very low dispersity, i.e. 1.10 or lower. For visualization, the SEC traces of these polymers are shown in Figure 1 and 2. It can clearly be seen that the present invention provides polymers with a very narrow molecular weight distribution.

Preparation and purity of the polymers of the present invention

Figure 4 shows the $^1$H NMR spectrum of $\alpha$-DMPEO-P(EG$_{0.70}$-*co*-DMP2GE$_{0.30}$), i.e., the polymer of Example 5 (entry c of Tables 1 and 2). The product is as obtained from Example 5, without further purification. As can be seen, the present method does not only provide polymers of narrow molecular weight distribution, but also very pure polymers without the need for laborious purification steps. This is an important feature for the use in pharmaceutical applications.

End-group fidelity

As can be seen from Table 2, the end-group fidelity for all polymers of the present invention is nearly 100%. Figure 3 shows the MALDI TOF mass spectrum of $\alpha$-DMPEO-P(EG$_{0.70}$-*co*-DMP2GE$_{0.30}$), i.e., the polymer of Example 5 (entry c of Tables 1 and 2) with potassium cation. The only peaks visible here are the peaks of the product molecule. There are no peaks from macromolecules with a different end group. This shows that end-group fidelity of the polymers of the present invention is very high. In addition, the spectrum confirms the narrow molecular weight distribution.

Solubility

Figure 5 shows the cloud point measurement of $\alpha$-DMPEO-P(EG$_{0.80}$-*co*-DMP2GE$_{0.20}$) and $\alpha$-DMPEO-P(EG$_{0.70}$-*co*-DMP2GE$_{0.30}$), i.e., the polymers of Example 5 (entry b and c of Tables 1 and 2). The cloud point determines the temperature at which immiscibility in water (and therefore in aqueous solution) is observed upon heating. As an example, for the copolymer with 30% DPM2GE (entry c of Tables 1 and 2), the cloud point can be observed at 94 °C, showing excellent water solubility of the polymers of the present invention. The homopolymer PEG shows a cloud point of ca. 100°C. For copolymers of <30 mol% glycidyl ether monomers with ether side chains incorporation no cloud point was detected in water at temperatures ranging from 0 to 99 °C. Thus, the ether side chains of glycidyl ether monomer repeating units hardly affect water solubility, which is a feature of the similar structure of polyethylene glycol and polymers of the present invention. This is an important feature of the polymers of the present invention as it shows that they can substitute PEG in aqueous systems without causing problems with phase separation or even precipitation.

Crystallinity

Table 4 shows thermal properties of some of the polymers of Tables 1 and 2:

Table 4: Thermal properties in bulk

| Sample | $T_g$ [°C] | $T_m$ [°C] | $\Delta H_{PEG}$ [J/g] | $X_{c,PEG}$ [%] |
|---|---|---|---|---|
| a | -65 | 16 | 48.3 | 24 |
| b | -66 | N/O | N/O | N/O |
| c | -66 | N/O | N/O | N/O |
| d | -67 | N/O | N/O | N/O |

$T_g$ = Glass transition temperature; $T_m$ = Melting point; = Enthalpy of fusion; $X_c$ = Crystallinity (all measurements by DSC).

As can be seen, crystallinity of the inventive polymers is low and can be completely interrupted by the incorporation of glycidyl ether monomers with ether side chains in the polymer structure. This prevents for example accumulation of the polymers in the kidney or in the liver.

Immunogenicity: Comparison of scPEGs with mPEG and P(EG-co-GME)

Table 5: ELISA results

| *Entry*/ (Example) | *Sample* | *Relative APA Affinity* |
|---|---|---|
| - | mPEG 5k | 1 |
| - | $\alpha$-BzO-P($EG_{0.76}$-*co*-$GME_{0.24}$) | 0.06 |
| b (5) | $\alpha$-DMPEO-P($EG_{0.80}$-*co*-$DMP2GE_{0.20}$) | 0.006 |
| c (5) | $\alpha$-DMPEO-P($EG_{0.70}$-*co*-$DMP2GE_{0.30}$) | 0.001 |

Relative APA affinities are calculated from the $IC_{50}$ values of the investigated polymers.

Figure 8 and Table 5 show the ELISA test results of entry b and c, P(EG-co-GME) and of commercially available mPEG 5k. Figure 8 shows the function of the normalized absorption at a wavelength of 450 nm versus the log10 function of the polymer concentration in nanograms per ml, therefore illustrating the anti-PEG antibody interaction with the investigated polymer concentrations. Table 5 illustrates the APA affinity of the investigated scPEGs (entry b and c) and P(EG-co-GME) (prepared according to WO 2022/238532 A1, Example 2, Table 3, Entry d) relative to mPEG 5k. The ELISA data shows a strong influence of the sterically demanding side chains of scPEGs (it is important to note that the x-axis has a logarithmic scale). With increasing amount of scEO incorporated into the polyether structure, significantly higher polymer concentrations are necessary to observe interactions between the copolymer and the APA. Remarkably, a significant stronger hindrance of APA binding was observed for scPEGs in comparison to P(EG-co-GME) with similar amount of incorporated comonomer.

## Claims

1. Polyether polymers comprising monomer units that have a structure of formula (I):

$$-[CH_2\text{-}CHR\text{-}O]_n- \qquad (I),$$

wherein residues R are selected independently from each other from the group consisting of hydrogen, $R^1$ and $R^2$; wherein residues $R^1$ are selected independently of each other from the group consisting of

$$-CH_2\text{-}O\text{-}(CH_2\text{-}CH_2\text{-}O)_m\text{-}R^3,$$

$$-CH_2\text{-}O\text{-}(CH_2\text{-}CH_2\text{-}O)_x\text{-}CH(CH_2\text{-}O\text{-}(CH_2\text{-}CH_2\text{-}O)_a\text{-}R^3)(CH_2\text{-}O\text{-}(CH_2\text{-}CH_2\text{-}O)_b\text{-}R^3),$$

$$-CH_2\text{-}O\text{-}(CH_2\text{-}CH_2\text{-}O)_y\text{-}CH_2\text{-}CH(O\text{-}(CH_2\text{-}CH_2\text{-}O)_c\text{-}R^3)\text{-}CH_2\text{-}O\text{-}(CH_2\text{-}CH_2\text{-}O)_d\text{-}R^3,$$

wherein m are independently of each other 1, 2, 3, 4 or 5 and a, b, c, d, x and y are independently from each other 0 or 1;
wherein $R^2$ is $-CH_2-O-R^3$,
wherein residues $R^3$ are selected independently of each other from the group consisting of hydrogen, methyl, ethyl, *n*-propyl and *iso*-propyl;
and wherein at least 3 % of the residues R are residues $R^1$, based on the total number of all residues R in the polymer;
wherein the polyether polymers are either homopolymers or statistical copolymers and wherein at least 97 % of the monomer units of the polyether polymers are of formula (I), based on all monomer units of the polyether polymers;
wherein the polyether polymers have at least a number average molecular weight in the range of 1,000 to 50,000 g/mol as determined by MALDI-TOF MS and the dispersity of the polyether polymers is 1.15 or less as measured by size exclusion chromatography in DMF by calibration with PEG standards
**characterized in that**
the polyether polymers of Formula (I) are represented by the following formula [II]:

$$X-[CH_2CHR-O]_n-CH_2CHR-Y \qquad [II]$$

wherein R is as defined as in formula (I)
wherein X is selected from the group consisting of hydroxyl or -O-X',
wherein X' is selected from the group consisting of

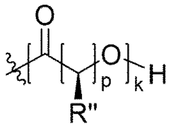
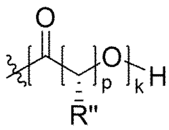
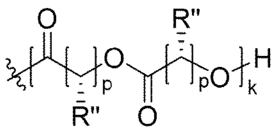

wherein R" = H or alkyl, alkenyl, aryl, alkoxymethyl, alkenyloxymethyl, aryloxymethyl, aminomethyl and thioalkoxymethyl and
p = 1, 2, 3, 4 or 5, and
k = 5-500;

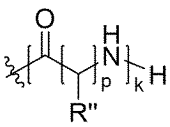
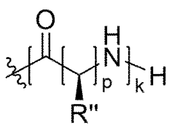
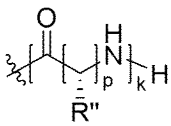

wherein R" = H or alkyl, alkenyl, aryl, alkoxymethyl, alkenyloxymethyl, aryloxymethyl, aminomethyl and thioalkoxymethyl, and
p = 1, 2, 3, 4 or 5 and
k = 5-500; and

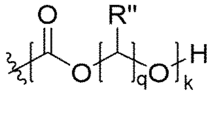

wherein R" = H or alkyl, alkenyl, aryl, alkoxymethyl, alkenyloxymethyl, aryloxymethyl, aminomethyl and thioalkoxymethyl
q = 3, 4 or 5 and
k = 5-500;
and

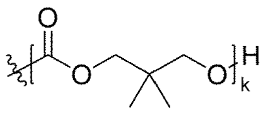

wherein k = 5-500; and
wherein Y is -Z-W, wherein
Z is -O-R'-O- and
W is $-CH_2CHR-(O-CH_2CHR-)_{n'}-V$;
wherein V is X or X';
wherein R' is selected from the group consisting of

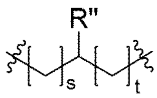

wherein
R" = H or alkyl, alkenyl, aryl, alkoxymethyl, alkenyloxymethyl, aryloxymethyl, aminomethyl and thioalkoxymethyl, and
s = 0-20
t = 0-20; and
further R' is selected from

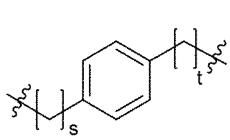

s = 0-20
t = 0-20

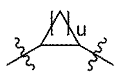

u = 1-10

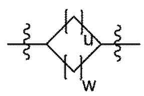

u = 1-10
w = 1-10

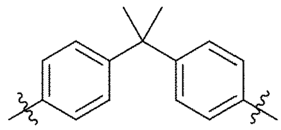

.

2. Polyether polymers according to claim 1 wherein at least 3 % of the residues R are hydrogen.

3. Polyether polymers according to any of claims 1 and 2 wherein at least 3 % of residues R are residues $R^2$.

4. Polyether polymers according to any of the preceding claims, wherein 3% to 95% of residues R are hydrogen and 3% to 95% of residues R are residues $R^1$.

5. Polyether polymers according to any of the preceding claims, wherein 10 to 50 %, preferably 18 to 40 % of residues R are residues $R^1$.

6. Polyether polymer according to any of claims 1 to 5, wherein V is selected from the group consisting of X' and -O-X'.

7. Polyether polymer according to any of claims 1 to 6, wherein X is -O-X'.

8. Polyether polymer according to any of claims 1 to 7, wherein end-group fidelity of the polymer in regard to group X and/or in regard to group Y is at least 95%.

9. Process for the preparation of polyether polymers according to any of claims 1 to 8 by anionic ring-opening copolymerization comprising the steps of:

- Providing an anion $An^-$,
- Adding at least one monomer of the formula

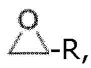

where in R is as defined in any of claims 1 to 5, in the amounts as described in any of claims 1 to 5 and adding optionally further monomers,

- Allowing the polymerization to proceed at a temperature in the range of -10 to 90 °C.

**10.** Conjugate, comprising a polyether polymer and a substrate, wherein the polyether polymer comprises monomer units that have a structure of formula (I):

$$-[CH_2-CHR-O]_n- \qquad (I),$$

wherein residues R are selected independently from each other from the group consisting of hydrogen, $R^1$ and $R^2$; wherein residues $R^1$ are selected independently of each other from the group consisting of

$$-CH_2-O-(CH_2-CH_2-O)_m-R^3,$$

$$-CH_2-O-(CH_2-CH_2-O)_x-CH(CH_2-O-(CH_2-CH_2-O)_a-R^3)(CH_2-O-(CH_2-CH_2-O)_b-R^3),$$

$$-CH_2-O-(CH_2-CH_2-O)_y-CH_2-CH(O-(CH_2-CH_2-O)_c-R^3)-CH_2-O-(CH_2-CH_2-O)_4-R^3,$$

wherein m are independently of each other 1, 2, 3, 4 or 5 and a, b, c, d, x and y, are independently from each other 0 or 1;
wherein $R^2$ is $-CH_2-O-R^3$,
wherein residues $R^3$ are selected independently of each other from the group consisting of hydrogen, methyl, ethyl, *n*-propyl and *iso*-propyl;
and wherein at least 3 % of the residues R are residues $R^1$, based on the total number of all residues R in the polymer; and preferably the polyether polymer is a polyether polymer according to any of claims 1 to 8 or a polyether polymer prepared by a process according to claim 9.

**11.** Conjugate according to claim 10, wherein the substrate is selected from the groups consisting of low molecular weight drugs, nanocarriers, liposomal structures, peptides, polypeptides, proteins, glycoproteins, polynucleotides, polysaccharides, lipid structures, liposomes, surfaces and interfaces.

**12.** Use of polyether polymers for the preparation of a conjugate of the polyether polymers with a bioactive compound, wherein the polyether polymer comprises monomer units that have a structure of formula (I):

$$-[CH_2-CHR-O]_n- \qquad (I),$$

wherein residues R are selected independently from each other from the group consisting of hydrogen, $R^1$ and $R^2$; wherein residues $R^1$ are selected independently of each other from the group consisting of

$$-CH_2-O-(CH_2-CH_2-O)_m-R^3,$$

$$-CH_2-O-(CH_2-CH_2-O)_x-CH(CH_2-O-(CH_{2_}CH_2-O)a-R^3)(CH_2-O-(CH_2-CH_2-O)_b-R^3),$$

$$-CH_2-O-(CH_2-CH_2-O)_y-CH_2-CH(O-(CH_2-CH_2-O)_c-R^3)-CH_2-O-(CH_2-CH_2-O)_d-R^3,$$

wherein m are independently of each other 1, 2, 3, 4 or 5 and a, b, c, d, x and y, are independently from each other 0 or 1;
wherein $R^2$ is $-CH_2-O-R^3$,
wherein residues $R^3$ are selected independently of each other from the group consisting of hydrogen, methyl, ethyl, *n*-propyl and *iso*-propyl;
and wherein at least 3 % of the residues R are residues $R^1$, based on the total number of all residues R in the polymer; and preferably the polyether polymer is a polyether polymer according to any of claims 1 to 8 or a polyether polymer prepared by a process according to claim 9.

**13.** Use of polyether polymers for the preparation of conjugated lipids for use in vaccines, in particular based on lipid nanoparticles, wherein these nanoparticles are preferably nanoparticles, as used against COVID-19 and wherein the polyether polymers comprise monomer units that have a structure of formula (I):

$$-[CH_2-CHR-O]_n- \qquad (I),$$

wherein residues R are selected independently from each other from the group consisting of hydrogen, $R^1$ and $R^2$; wherein residues $R^1$ are selected independently of each other from the group consisting of

$-CH_2-O-(CH_2-CH_2-O)_m-R^3$,

$-CH_2-O-(CH_2-CH_2-O)_x-CH(CH_2-O-(CH_{2-}CH_2-O)a-R^3)(CH_2-O-(CH_2-CH_2-O)_b-R^3)$,

$-CH_2-O-(CH_2-CH_2-O)_y-CH_2-CH(O-(CH_2-CH_2-O)_c-R^3)-CH_2-O-(CH_2-CH_2-O)_d-R^3$,

wherein m are independently of each other 1, 2, 3, 4 or 5 and a, b, c, d, x and y, are independently from each other 0 or 1;
wherein $R^2$ is $-CH_2-O-R^3$,
wherein residues $R^3$ are selected independently of each other from the group consisting of hydrogen, methyl, ethyl, *n*-propyl and *iso*-propyl;

and wherein at least 3 % of the residues R are residues $R^1$, based on the total number of all residues R in the polymer; and preferably the polyether polymer is a polyether polymer according to any of claims 1 to 8 or a polyether polymer prepared by a process according to claim 9.

## Patentansprüche

**1.** Polyetherpolymere umfassend Monomereinheiten, die eine Struktur der Formel (I) aufweisen:

$$-[CH_2-CH_R-O]_n- \qquad (I),$$

wobei die Reste R unabhängig voneinander aus der Gruppe bestehend aus Wasserstoff, $R^1$ und $R^2$ ausgewählt sind;
wobei die Reste $R^1$ unabhängig voneinander aus der Gruppe bestehend aus

$-CH_2-O-(CH_2-CH_2-O)_m-R^3$,

$-CH_2-O-(CH_2-CH_2-O)_x-CH(CH_2-O-(CH_2-CH_2-O)_a-R^3)(CH_2-O-(CH_2-CH_2-O)_b-R^3)$,

$-CH_2-O-(CH_2-CH_2-O)_y-CH_2-CH(O-(CH_2-CH_2-O)_c-R_3)-CH_2-O-(CH_2-CH_2-O)_d-R^3$,

ausgewählt sind, wobei m unabhängig voneinander 1, 2, 3, 4 oder 5 sind und a, b, c, d, x und y unabhängig voneinander 0 oder 1 sind;
wobei $R^2$ ist, $-CH_2-O-R^3$,
wobei die Reste $R^3$ unabhängig voneinander aus der Gruppe bestehend aus Wasserstoff, Methyl, Ethyl, *n*-Propyl und *iso*-Propyl ausgewählt sind;
und wobei mindestens 3% der Reste R Reste $R^1$ sind, bezogen auf die Gesamtzahl aller Reste R in dem Polymer;
wobei die Polyetherpolymere entweder Homopolymere oder statistische Copolymere sind und wobei mindestens 97% der Monomereinheiten der Polyetherpolymere der Formel (I) entsprechen, bezogen auf alle Monomereinheiten der Polyetherpolymere;
wobei die Polyetherpolymere mindestens ein zahlenmittleres Molekulargewicht im Bereich von 1.000 bis 50.000 g/mol aufweisen, bestimmt durch MALDI-TOF MS, und die Dispersität der Polyetherpolymere 1,15 oder weniger beträgt, gemessen durch Größenausschlusschromatographie in DMF durch Kalibrierung mit PEG-Standards **dadurch gekennzeichnet, dass**

die Polyetherpolymere der Formel (I) durch die folgende Formel [II] dargestellt werden:

$$X\text{-}[CH_2CHR\text{-}O]_n\text{-}CH_2CHR\text{-}Y \qquad [II]$$

wobei R wie in Formel (I) definiert ist
wobei X aus der Gruppe bestehend aus Hydroxyl oder -O-X’ ausgewählt ist,
wobei X’ ausgewählt ist aus der Gruppe bestehend aus

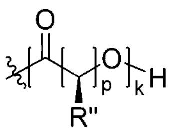 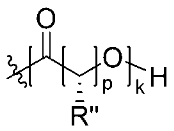 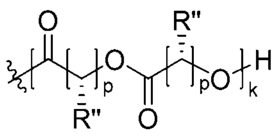

wobei R" = H oder Alkyl, Alkenyl, Aryl, Alkoxymethyl, Alkenyloxymethyl, Aryloxymethyl, Aminomethyl und Thioalkoxymethyl ist und
p = 1, 2, 3, 4 oder 5, und
k = 5-500;

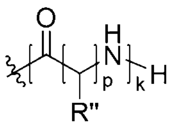 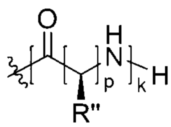 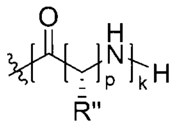

wobei R" = H oder Alkyl, Alkenyl, Aryl, Alkoxymethyl, Alkenyloxymethyl, Aryloxymethyl, Aminomethyl und Thioalkoxymethyl ist und
p = 1, 2, 3, 4 oder 5, und
k = 5-500; und

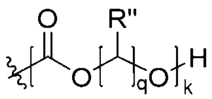

wobei R" = H oder Alkyl, Alkenyl, Aryl, Alkoxymethyl, Alkenyloxymethyl, Aryloxymethyl, Aminomethyl und Thioalkoxymethyl ist
q = 3, 4 oder 5 und
k = 5-500;
und

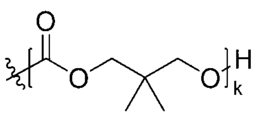

wobei k = 5-500; und
wobei Y ist -Z-W, wobei
Z ist -O-R’-O- und
W ist $-CH_2CHR-(O-CH_2CHR-)_{n'}-V$;
wobei V is X or X’;
wobei R’ ausgewählt ist aus der Gruppe bestehend aus

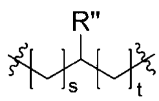

wobei
R" = H oder Alkyl, Alkenyl, Aryl, Alkoxymethyl, Alkenyloxymethyl, Aryloxymethyl, Aminomethyl und Thioalkoxymethyl, und
s = 0-20
t = 0-20; und
ferner ist R' ausgewählt aus

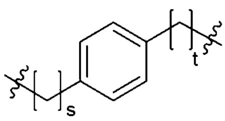

s = 0-20
t = 0-20

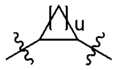

u = 1-10

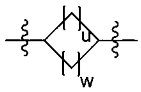

u = 1-10
w = 1-10

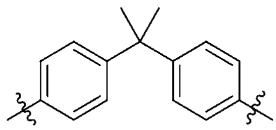

.

**2.** Polyetherpolymere nach Anspruch 1, wobei mindestens 3% der Reste R Wasserstoff sind.

**3.** Polyetherpolymere nach einem der Ansprüche 1 und 2, wobei mindestens 3% der Reste R Reste $R^2$ sind.

**4.** Polyetherpolymere nach einem der vorhergehenden Ansprüche, wobei 3% bis 95% der Reste R Wasserstoff sind und 3% bis 95% der Reste R Reste $R^1$ sind.

**5.** Polyetherpolymere nach einem der vorhergehenden Ansprüche, wobei 10 bis 50%, vorzugsweise 18 bis 40% der Reste R Reste $R^1$ sind.

**6.** Polyetherpolymer nach einem der Ansprüche 1 bis 5, wobei V aus der Gruppe bestehend aus X' und -O-X' ausgewählt ist.

**7.** Polyetherpolymer nach einem der Ansprüche 1 bis 6, wobei X ist -O-X'.

**8.** Polyetherpolymer nach einem der Ansprüche 1 bis 7, wobei die Endgruppentreue des Polymers bezüglich der Gruppe X und/oder bezüglich der Gruppe Y mindestens 95% beträgt.

**9.** Verfahren zur Herstellung von Polyetherpolymeren nach einem der Ansprüche 1 bis 8 durch anionische Ringöffnungscopolymerisation umfassend die Schritte:

- Bereitstellen eines Anions $An^-$,
- Zugabe mindestens eines Monomers der Formel

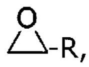,

wobei R wie in einem der Ansprüche 1 bis 5 definiert ist, in den in einem der Ansprüche 1 bis 5 beschriebenen Mengen und gegebenenfalls Zugabe weiterer Monomere,
- Durchführung der Polymerisation bei einer Temperatur im Bereich von -10 bis 90°C.

**10.** Konjugat, umfassend ein Polyetherpolymer und ein Substrat, wobei das Polyetherpolymer Monomereinheiten umfasst, die eine Struktur der Formel (I) aufweisen:

$$-[CH_2\text{-}CHR\text{-}O]_n- \qquad (I),$$

wobei die Reste R unabhängig voneinander aus der Gruppe bestehend aus Wasserstoff, $R^1$ und $R^2$ ausgewählt sind;
wobei die Reste $R^1$ unabhängig voneinander aus der Gruppe bestehend aus

$$-CH_2\text{-}O\text{-}(CH_2\text{-}CH_2\text{-}O)_m\text{-}R^3,$$

$-CH_2-O-(CH_2-CH_2-O)_x-CH(CH_2-O-(CH_2-CH_2-O)_a-R^3)(CH_2-O-(CH_2-CH_2-O)_b-R^3),$

$-CH_2-O-(CH_2-CH_2-O)_y-CH_2-CH(O-(CH_2-CH_2-O)_c-R^3)-CH_2-O-(CH_2-CH_2-O)_d-R^3,$

ausgewählt sind, wobei m unabhängig voneinander 1, 2, 3, 4 oder 5 sind und a, b, c, d, x und y unabhängig voneinander 0 oder 1 sind;
wobei $R^2$ ist, $-CH_2-O-R^3$,
wobei die Reste $R^3$ unabhängig voneinander aus der Gruppe bestehend aus Wasserstoff, Methyl, Ethyl, *n*-Propyl und *iso*-Propyl ausgewählt sind;
und wobei mindestens 3% der Reste R Reste $R^1$ sind, bezogen auf die Gesamtzahl aller Reste R in dem Polymer; und vorzugsweise ist das Polyetherpolymer ein Polyetherpolymer nach einem der Ansprüche 1 bis 8 oder ein Polyetherpolymer, das durch ein Verfahren nach Anspruch 9 hergestellt wurde.

**11.** Konjugat nach Anspruch 10, wobei das Substrat aus der Gruppe bestehend aus niedermolekularen Arzneimitteln, Nanoträgern, liposomalen Strukturen, Peptiden, Polypeptiden, Proteinen, Glykoproteinen, Polynukleotiden, Polysacchariden, Lipidstrukturen, Liposomen, Oberflächen und Grenzflächen ausgewählt ist.

**12.** Verwendung von Polyetherpolymeren zur Herstellung eines Konjugats der Polyetherpolymere mit einer bioaktiven Verbindung, wobei das Polyetherpolymer Monomereinheiten umfasst, die eine Struktur der Formel (I) aufweisen:

$$-[CH_2-CHR-O]_n- \qquad (I),$$

wobei die Reste R unabhängig voneinander aus der Gruppe bestehend aus Wasserstoff, $R^1$ und $R^2$ ausgewählt sind;
wobei die Reste $R^1$ unabhängig voneinander aus der Gruppe bestehend aus

$-CH_2-O-(CH_2-CH_2-O)_m-R^3,$

$-CH_2-O-(CH_2-CH_2-O)_x-CH(CH_2-O-(CH_2-CH_2-O)_a-R^3)(CH_2-O-(CH_2-CH_2-O)_b-R^3),$

$-CH_2-O-(CH_2-CH_2-O)_y-CH_2-CH(O-(CH_2-CH_2-O)_c-R^3)-CH_2-O-(CH_2-CH_2-O)_d-R^3$

ausgewählt sind,
wobei m unabhängig voneinander 1, 2, 3, 4 oder 5 sind und a, b, c, d, x und y unabhängig voneinander 0 oder 1 sind;
wobei $R^2$ ist $-CH_2-O-R^3$,
wobei die Reste $R^3$ unabhängig voneinander aus der Gruppe bestehend aus Wasserstoff, Methyl, Ethyl, *n*-Propyl und *iso*-Propyl ausgewählt sind;
und wobei mindestens 3% der Reste R Reste $R^1$ sind, bezogen auf die Gesamtzahl aller Reste R in dem Polymer; und vorzugsweise ist das Polyetherpolymer ein Polyetherpolymer nach einem der Ansprüche 1 bis 8 oder ein Polyetherpolymer, das durch ein Verfahren nach Anspruch 9 hergestellt wurde.

**13.** Verwendung von Polyetherpolymeren zur Herstellung konjugierter Lipide für die Verwendung in Impfstoffen, insbesondere basierend auf Lipid-Nanopartikeln, wobei diese Nanopartikel vorzugsweise Nanopartikel sind, wie sie gegen COVID-19 verwendet werden, und wobei die Polyetherpolymere Monomereinheiten umfassen, die eine Struktur der Formel (I) aufweisen:

$$-[CH_2-CHR-O]_n- \qquad (I),$$

wobei die Reste R unabhängig voneinander aus der Gruppe bestehend aus Wasserstoff, $R^1$ und $R^2$ ausgewählt sind;
wobei die Reste $R^1$ unabhängig voneinander aus der Gruppe bestehend aus

$-CH_2-O-(CH_2-CH_2-O)_m-R^3,$

$-CH_2-O-(CH_2-CH_2-O)_x-CH(CH_2-O-(CH_2-CH_2-O)_a-R^3)(CH_2-O-(CH_2-CH_2-O)_b-R^3)$,

$-CH_2-O-(CH_2-CH_2-O)_y-CH_2-CH(O-(CH_2-CH_2-O)_c-R^3)-CH_2-O-(CH_2-CH_2-O)_d-R^3$,

ausgewählt sind, wobei m unabhängig voneinander 1, 2, 3, 4 oder 5 sind und a, b, c, d, x und y unabhängig voneinander 0 oder 1 sind;
wobei $R^2$ ist $-CH_2-O-R^3$,
wobei die Reste $R^3$ unabhängig voneinander aus der Gruppe bestehend aus Wasserstoff, Methyl, Ethyl, *n*-Propyl und *iso*-Propyl ausgewählt sind; und
wobei mindestens 3% der Reste R Reste $R^1$ sind, bezogen auf die Gesamtzahl aller Reste R in dem Polymer;
und wobei vorzugsweise das Polyetherpolymer ein Polyetherpolymer nach einem der Ansprüche 1 bis 8 oder ein Polyetherpolymer, das durch ein Verfahren nach Anspruch 9 hergestellt wurde, ist.

## Revendications

**1.** Polymères polyéthers comprenant des unités monomères ayant une structure de formule (I):

$$-[CH_2-CHR-O]_n- \qquad (I),$$

dans laquelle les résidus R sont choisis indépendamment les uns des autres dans le groupe constitué par l'hydrogène, $R^1$ et $R^2$;
dans laquelle les résidus $R^1$ sont choisis indépendamment les uns des autres dans le groupe constitué par

$-CH_2-O-(CH_2-CH_2-O)_m-R^3$,

$-CH_2-O-(CH_2-CH_2-O)_x-CH(CH_2-O-(CH_2-CH_2-O)_a-R^3)(CH_2-O-(CH_2-CH_2-O)_b-R^3)$,

$-CH_2-O-(CH_2-CH_2-O)_y-CH_2-CH(O-(CH_2-CH_2-O)_c-R^3)-CH_2-O-(CH_2-CH_2-O)_d-R^3$,

dans
laquelle m sont indépendamment les uns des autres 1, 2, 3, 4 ou 5 et a, b, c, d, x
et y sont indépendamment les uns des autres 0 ou 1 ;
dans laquelle $R^2$ est $-CH_2-O-R^3$,
dans laquelle les résidus $R^3$ sont choisis indépendamment les uns des autres dans le groupe constitué par l'hydrogène, le méthyle, l'éthyle, le *n*-propyle et l'*iso*-propyle ;
et dans laquelle au moins 3% des résidus R sont des résidus $R^1$, par rapport au nombre total de tous les résidus R dans le polymère ;
dans laquelle les polymères polyéthers sont soit des homopolymères soit des copolymères statistiques et dans laquelle au moins 97% des unités monomères des polymères polyéthers sont de formule (I), par rapport à toutes les unités monomères des polymères polyéthers ;
dans laquelle les polymères polyéthers ont au moins une masse moléculaire moyenne en nombre dans la gamme de 1 000 à 50 000 g/mol telle que déterminée par MALDI-TOF MS et la dispersité des polymères polyéthers est de 1,15 ou moins telle que mesurée par chromatographie d'exclusion stérique dans le DMF par étalonnage avec des standards PEG
**caractérisés en ce que**
les polymères polyéthers de formule (I) sont représentés par la formule suivante [II] :

$$X-[CH_2CHR-O]_n-CH_2CHR-Y \qquad [II]$$

dans laquelle R est tel que défini dans la formule (I)
dans laquelle X est choisi dans le groupe constitué par l'hydroxyle ou -O-X',
dans laquelle X' est choisi dans le groupe constitué par

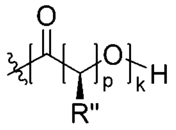 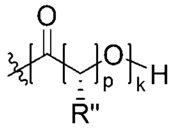 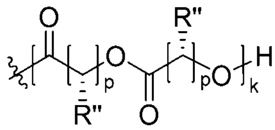

dans laquelle R" = H ou alkyle, alcényle, aryle, alcoxyméthyle, alcényloxyméthyle, aryloxyméthyle, aminométhyle et thioalcoxyméthyle et
p = 1, 2, 3, 4 or 5, et
k = 5-500;

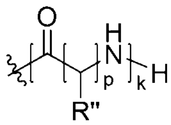 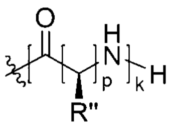 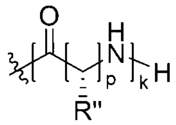

dans laquelle R" = H ou alkyle, alcényle, aryle, alcoxyméthyle, alcényloxyméthyle, aryloxyméthyle, aminométhyle et thioalcoxyméthyle, et
p = 1, 2, 3, 4 ou 5 et
k = 5-500 ; et

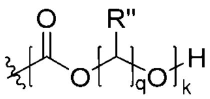

dans laquelle R" = H ou alkyle, alcényle, aryle, alcoxyméthyle, alcényloxyméthyle, aryloxyméthyle, aminométhyle et thioalcoxyméthyle
q = 3, 4 ou 5 et
k = 5-500 ;
et

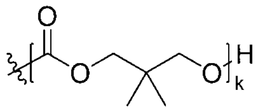

dans laquelle k = 5-500; et
dans laquelle Y est -Z-W, dans laquelle
Z est -O-R'-O- et
W est $-CH_2CHR-(O-CH_2CHR-)_{n'}-V$;
dans laquelle V est X ou X';
dans laquelle R' est choisi dans le groupe composé de

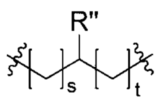

dans laquelle R" = H ou alkyle, alcényle, aryle, alcoxyméthyle, alcényloxyméthyle, aryloxyméthyle, aminométhyle et thioalcoxyméthyle, et
s = 0-20
t = 0-20; et
R' est sélectionné parmi

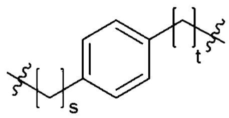

s = 0-20
t = 0-20

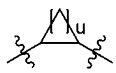

u = 1-10

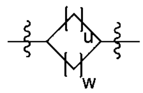

u = 1-10
w = 1-10

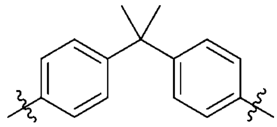

.

2. Polymères de polyéther selon la revendication 1, dans lesquels au moins 3 % des résidus R sont de l'hydrogène.

3. Polymères de polyéther selon l'une quelconque des revendications 1 et 2, dans lesquels au moins 3 % des résidus R sont des résidus $R^2$.

4. Polymères de polyéther selon l'une quelconque des revendications précédentes, dans lequel 3 % à 95 % des résidus R sont de l'hydrogène et 3 % à 95 % des résidus R sont des résidus $R^1$.

5. Polymères polyéthers selon l'une quelconque des revendications précédentes, dans lesquels 10 à 50 %, de préférence 18 à 40 % des résidus R sont des résidus $R^1$.

6. Polymère de polyéther selon l'une quelconque des revendications 1 à 5, dans lequel V est choisi dans le groupe constitué de X' et -O-X'.

7. Polymère de polyéther selon l'une quelconque des revendications 1 à 6, dans laquelle X est -O-X'.

8. Polymère de polyéther selon l'une quelconque des revendications 1 à 7, dans lequel la fidélité du polymère au groupe final par rapport au groupe X et/ou par rapport au groupe Y est d'au moins 95 %.

9. Procédé de préparation de polymères de polyéther selon l'une quelconque des revendications 1 à 8 par copolymérisation anionique à ouverture de cycle, comprenant les étapes consistant à :

- Fournir un anion $An^-$,
- Ajout d'au moins un monomère de la formule

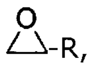

dans laquelle R est tel que défini dans l'une quelconque des revendications 1 à 5, dans les quantités décrites dans l'une quelconque des revendications 1 à 5 et en ajoutant éventuellement d'autres monomères,
- Permettre à la polymérisation de se dérouler à une température comprise entre -10 et 90 °C.

10. Conjugué, comprenant un polymère de polyéther et un substrat, dans lequel le polymère de polyéther comprend des unités monomères qui ont une structure de formule (I):

$$-[CH_2\text{-}CHR\text{-}O]_n- \qquad (I),$$

dans laquelle les résidus R sont choisis indépendamment les uns des autres dans le groupe constitué par l'hydrogène, $R^1$ et $R^2$;
dans lequel les résidus $R^1$ sont choisis indépendamment les uns des autres dans le groupe constitué par

$$-CH_2\text{-}O\text{-}(CH_2\text{-}CH_2\text{-}O)_m\text{-}R^3,$$

$$-CH_2\text{-}O\text{-}(CH_2\text{-}CH_2\text{-}O)_x\text{-}CH(CH_2\text{-}O\text{-}(CH_2\text{-}CH_2\text{-}O)_a\text{-}R^3)(CH_2\text{-}O\text{-}(CH_2\text{-}CH_2\text{-}O)_b\text{-}R^3),$$

$$-CH_2\text{-}O\text{-}(CH_2\text{-}CH_2\text{-}O)_y\text{-}CH_2\text{-}CH(O\text{-}(CH_2\text{-}CH_2\text{-}O)_c\text{-}R^3)\text{-}CH_2\text{-}O\text{-}(CH_2\text{-}CH_2\text{-}O)_d\text{-}R^3,$$

où m
sont indépendamment l'un de l'autre 1, 2, 3, 4 ou 5 et a, b, c, d, x et
y, sont indépendamment l'un de l'autre 0 ou 1;
dans laquelle $R^2$ est $-CH_2-O-R^3$,
dans laquelle les résidus $R^3$ sont choisis indépendamment les uns des autres dans le groupe constitué de l'hydrogène, du méthyle, de l'éthyle, du n-propyle et de l'*iso*-propyle;
et dans laquelle au moins 3 % des résidus R sont des résidus $R^1$, sur la base du nombre total de tous les résidus R dans le polymère; et de préférence, le polymère polyéther est un polymère polyéther selon l'une quelconque des revendications 1 à 8 ou un polymère polyéther préparé par un procédé selon la revendication 9.

**11.** Conjugué selon la revendication 10, dans laquelle le substrat est choisi parmi les groupes constitués de médicaments de faible poids moléculaire, de nanotransporteurs, de structures liposomales, de peptides, de polypeptides, de protéines, de glycoprotéines, de polynucléotides, de polysaccharides, de structures lipidiques, de liposomes, de surfaces et d'interfaces.

**12.** Utilisation de polymères de polyéther pour la préparation d'un conjugué des polymères de polyéther avec un composé bioactif, dans laquelle le polymère de polyéther comprend des unités monomères qui ont une structure de formule (I) :

$$-[CH_2-CHR-O]_n- \qquad (I),$$

dans laquelle les résidus R sont choisis indépendamment les uns des autres dans le groupe constitué par l'hydrogène, $R^1$ et $R^2$ ;
dans laquelle les résidus $R^1$ sont choisis indépendamment les uns des autres dans le groupe constitué par

$$-CH_2-O-(CH_2-CH_2-O)_m-R^3,$$

$$-CH_2-O-(CH_2-CH_2-O)_x-CH(CH_2-O-(CH_2-CH_2-O)_a-R^3)(CH_2-O-(CH_2-CH_2-O)_b-R^3),$$

$$-CH_2-O-(CH_2-CH_2-O)_y-CH_2-CH(O-(CH_2-CH_2-O)_c-R^3)-CH_2-O-(CH_2-CH_2-O)_d-R^3,$$

où m
sont indépendamment l'un de l'autre 1, 2, 3, 4 ou 5 et a, b, c, d, x et
y, sont indépendamment l'un de l'autre 0 ou 1;
dans laquelle $R^2$ est $-CH_2-O-R^3$,
dans laquelle les résidus $R^3$ sont choisis indépendamment les uns des autres dans le groupe constitué de l'hydrogène, du méthyle, de l'éthyle, du n-propyle et de l'*iso*-propyle;
et dans laquelle au moins 3 % des résidus R sont des résidus $R^1$, sur la base du nombre total de tous les résidus R dans le polymère; et de préférence, le polymère polyéther est un polymère polyéther selon l'une quelconque des revendications 1 à 8 ou un polymère polyéther préparé par un procédé selon la revendication 9.

**13.** Utilisation de polymères de polyéther pour la préparation de lipides conjugués pour utilisation dans des vaccins, en particulier à base de nanoparticules lipidiques, dans laquelle ces nanoparticules sont de préférence des nanoparticules, telles qu'utilisées contre la COVID-19 et dans laquelle les polymères de polyéther comprennent des unités monomères ayant une structure de formule (I) :

$$-[CH_2-CHR-O]_n- \qquad (I),$$

dans laquelle les résidus R sont choisis indépendamment les uns des autres dans le groupe constitué par l'hydrogène, $R^1$ et $R^2$;
dans laquelle les résidus $R^1$ sont choisis indépendamment les uns des autres dans le groupe constitué par

$$-CH_2-O-(CH_2-CH_2-O)_m-R^3,$$

$$-CH_2-O-(CH_2-CH_2-O)_x-CH(CH_2-O-(CH_2-CH_2-O)_a-R^3)(CH_2-O-(CH_2-CH_2-O)_b-R^3),$$

$$-CH_2-O-(CH_2-CH_2-O)_y-CH_2-CH(O-(CH_2-CH_2-O)_c-R^3)-CH_2-O-(CH_2-CH_2-O)_d-R^3,$$

dans
dans laquelle m sont indépendamment l'un de l'autre 1, 2, 3, 4 ou 5 et a, b, c, d,
x et y sont indépendamment les uns de l'autre 0 ou 1;
dans laquelle $R^2$ est $-CH_2-O-R^3$,
dans laquelle les résidus $R^3$ sont choisis indépendamment les uns des autres dans le groupe constitué de l'hydrogène, du méthyle, de l'éthyle, du n-propyle et de l'*iso*-propyle;

et dans laquelle au moins 3 % des résidus R sont des résidus $R^1$, sur la base du nombre total de tous les résidus R dans le polymère ; et de préférence, le polymère polyéther est un polymère polyéther selon l'une quelconque des revendications 1 à 8 ou un polymère polyéther préparé par un procédé selon la revendication 9.

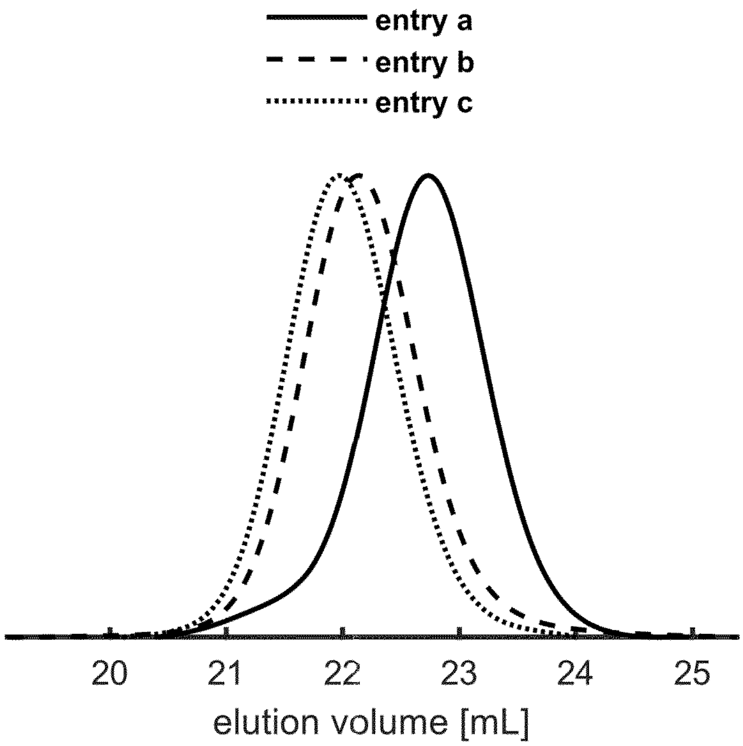


Figure 1

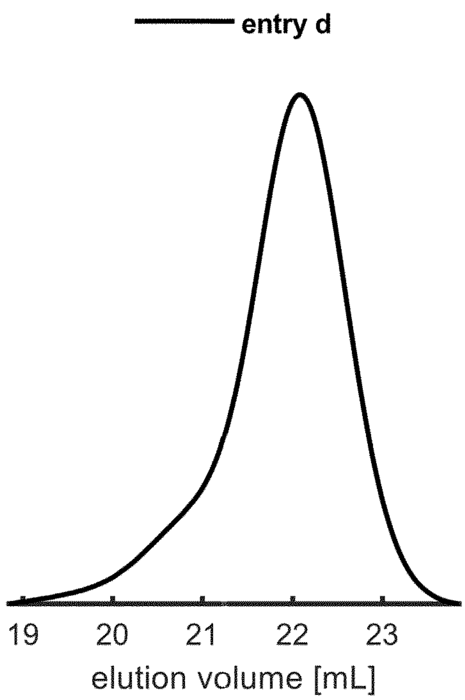


Figure 2

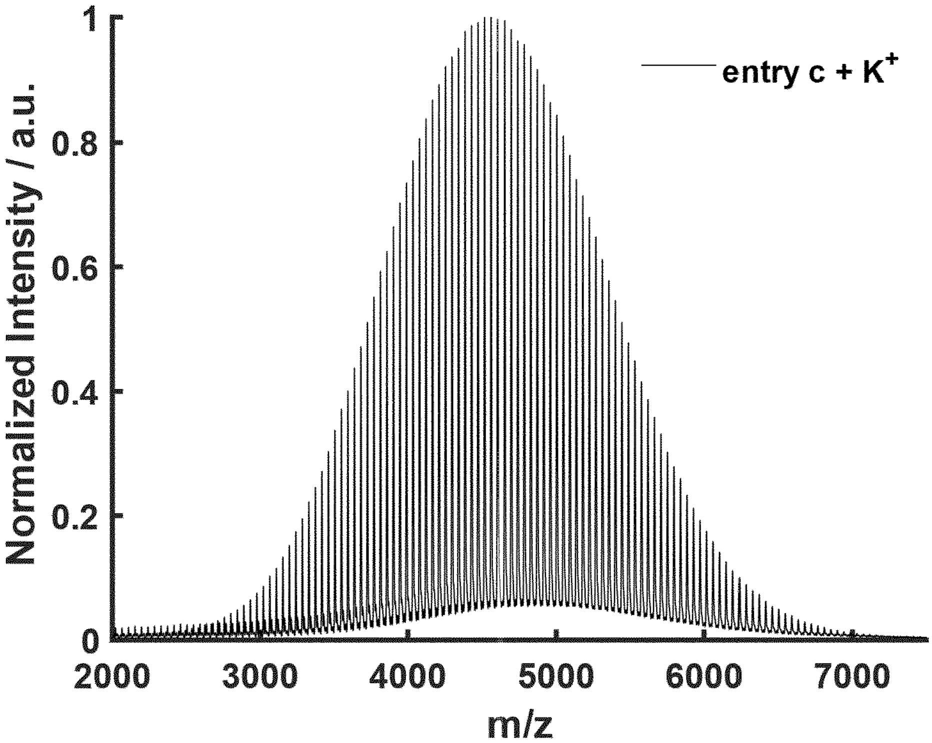
entry c + K+
Normalized Intensity / a.u.
1
0.8
0.6
0.4
0.2
0
2000
3000
4000
5000
6000
7000
m/z

Figure 3

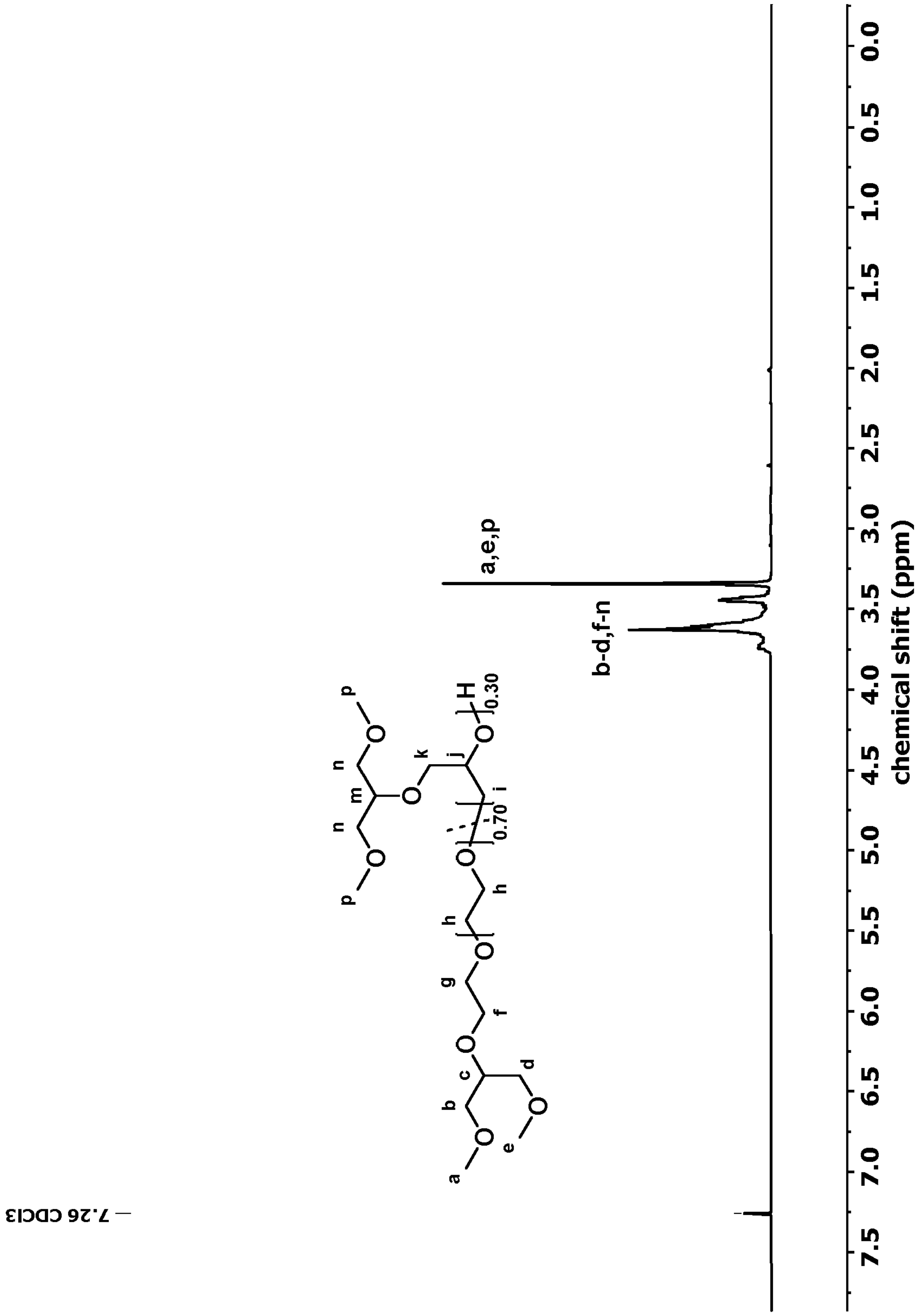
7.26 CDCl3
a,e,p
b-d,f-n
0.0
0.5
1.0
1.5
2.0
2.5
3.0
3.5
4.0
4.5
5.0
5.5
6.0
6.5
7.0
7.5
chemical shift (ppm)

Figure 4

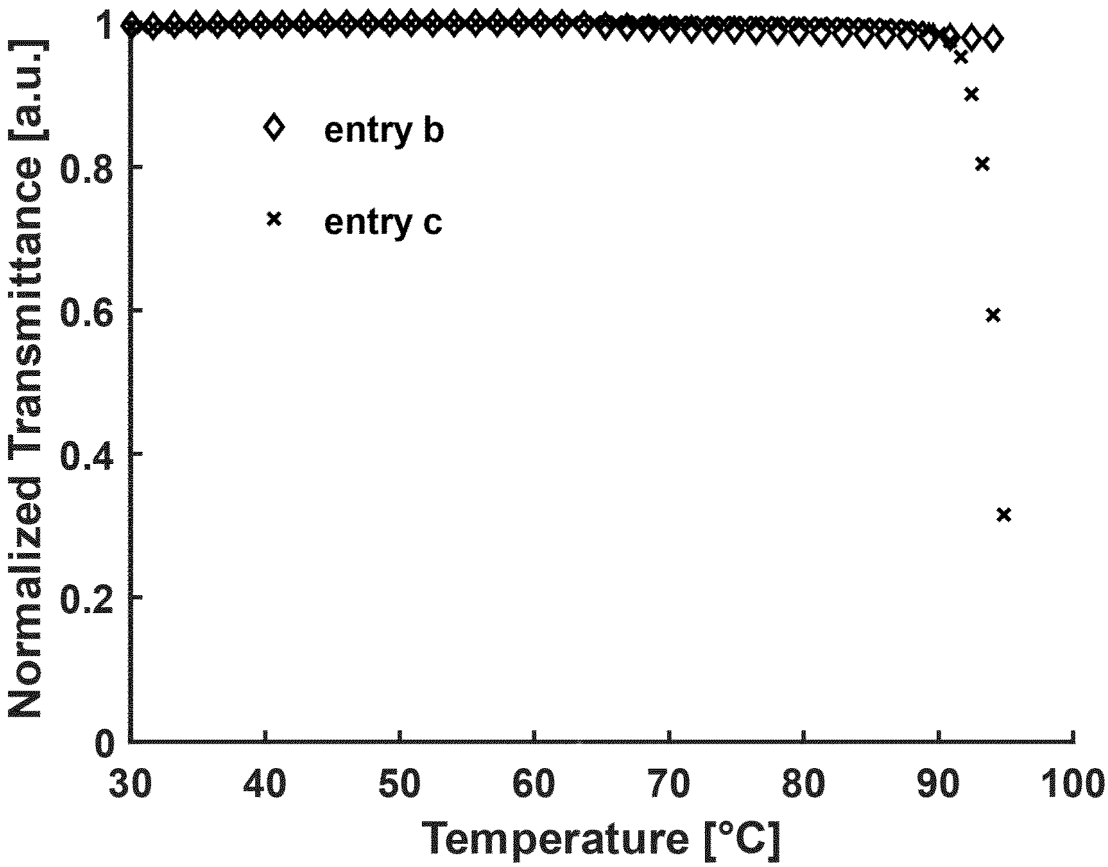
1
0.8
0.6
0.4
0.2
0
Normalized Transmittance [a.u.]
entry b
entry c
30
40
50
60
70
80
90
100
Temperature [°C]

Figure 5

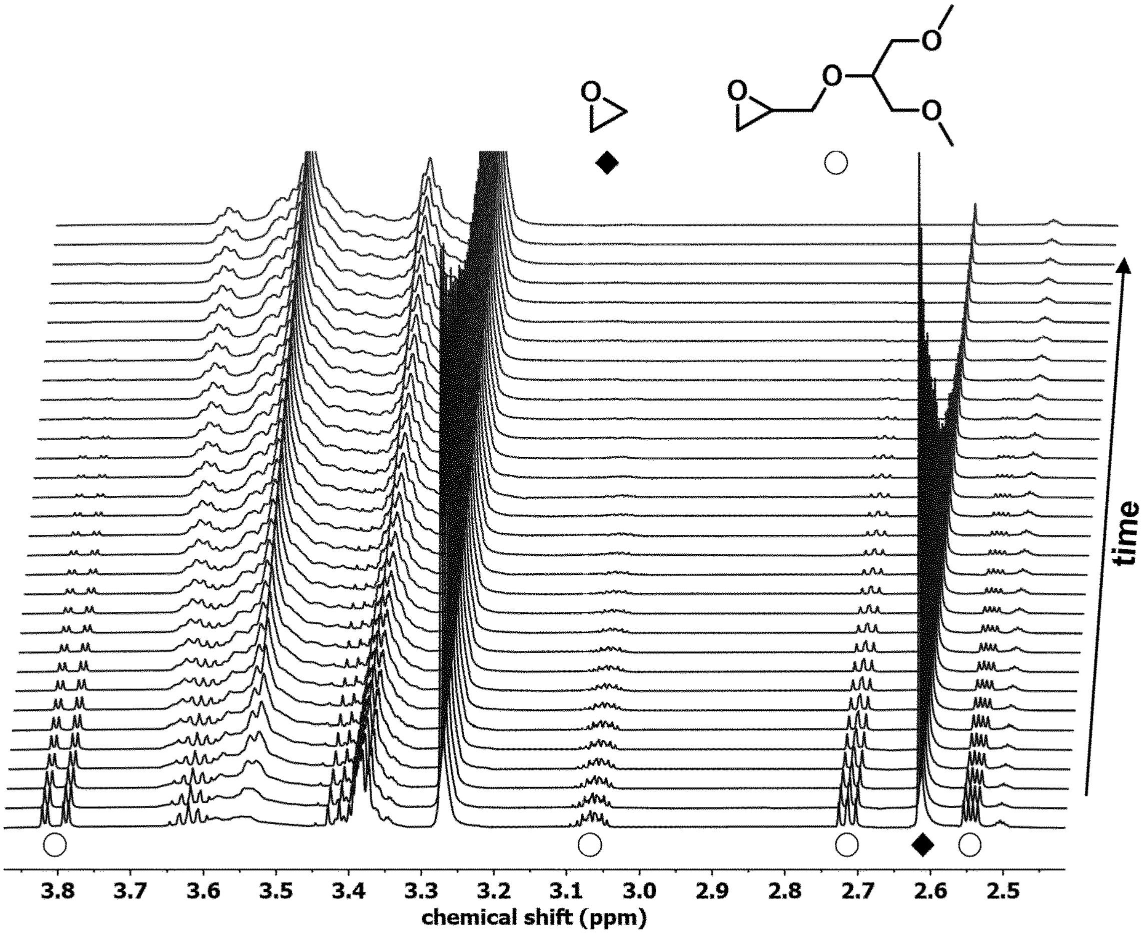


Figure 6

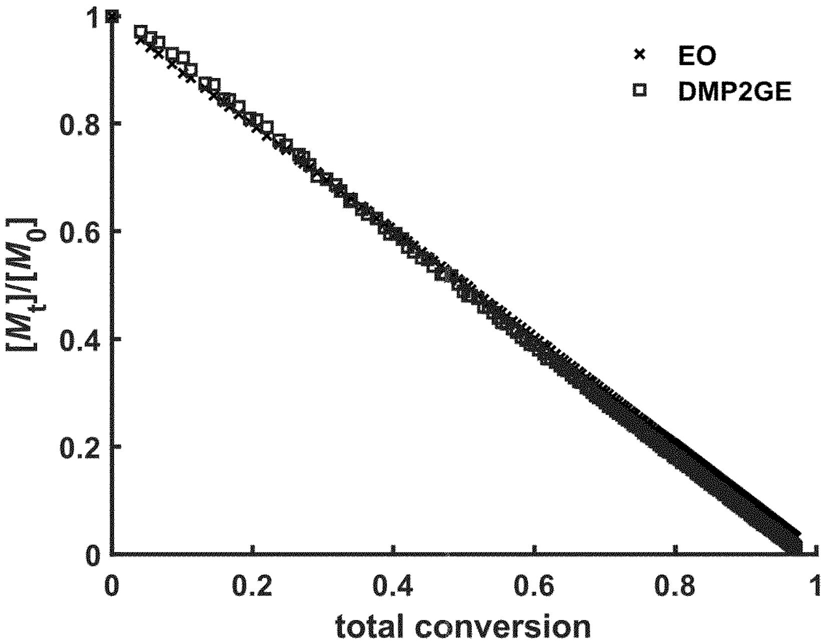


Figure 7

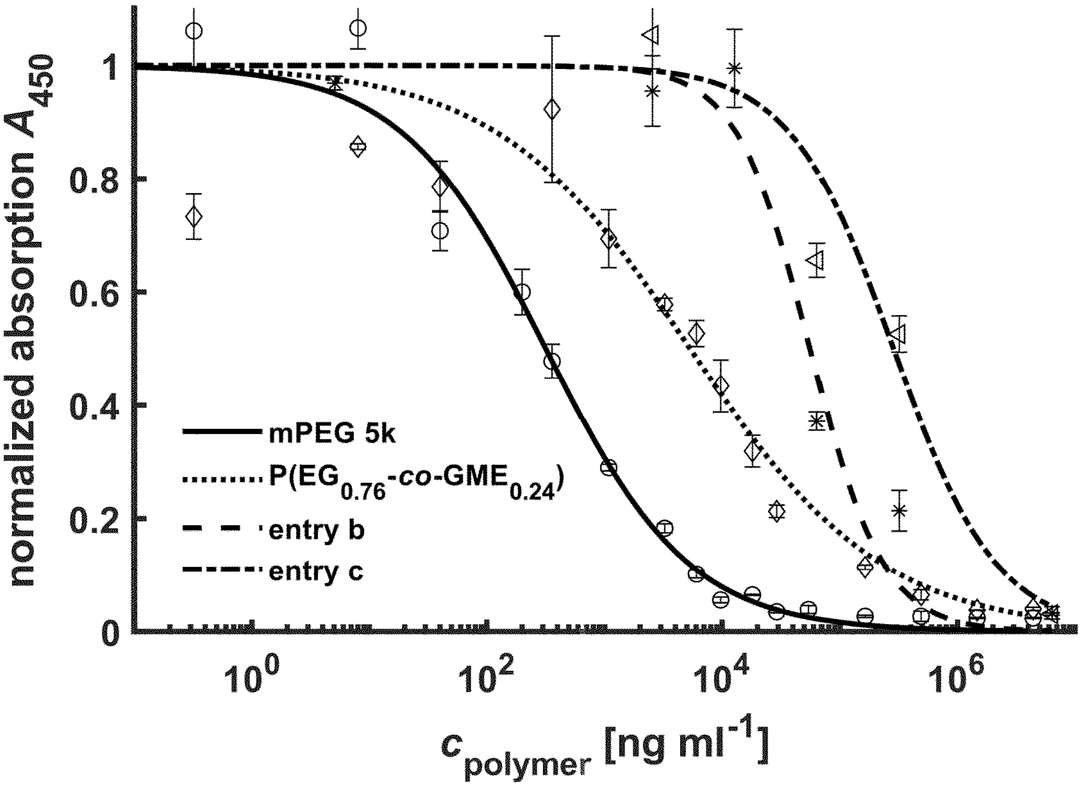


Figure 8

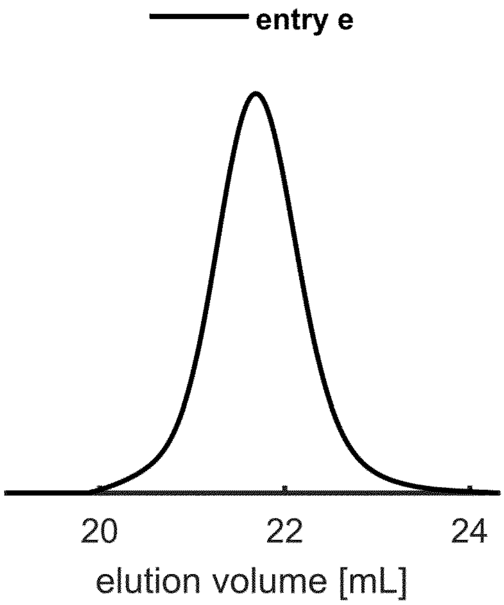


Figure 9

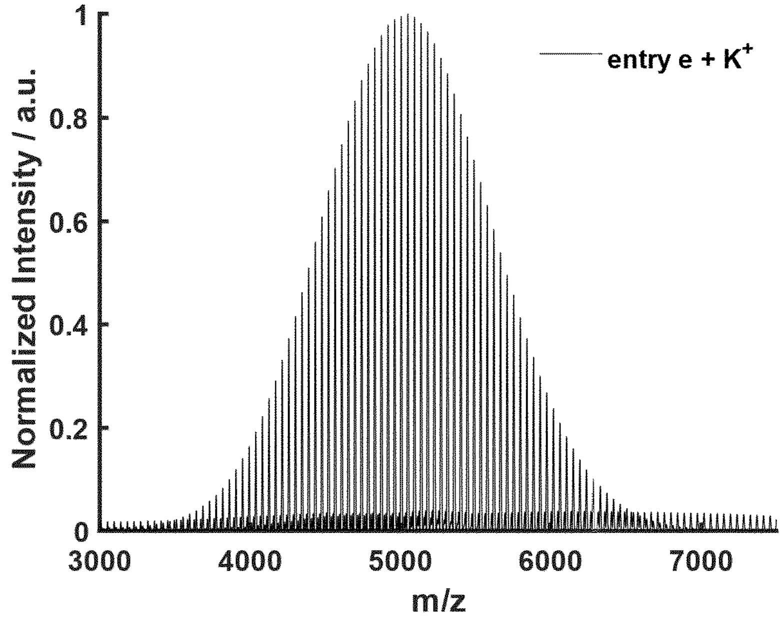
entry e + K+
Normalized Intensity / a.u.
1
0.8
0.6
0.4
0.2
0
3000
4000
5000
6000
7000
m/z

Figure 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8129330 B2
- US 20190015520 A1
- WO 2022238532 A1
- EP 22203748 A
- US 6162563 A


### Non-patent literature cited in the description


- **YAROVSKY et al.** Quantitative design rules for protein-resistant surface coatings using machine learning. *Scientific Rep*, 2019, vol. 9, 265
- **LAI et al.** Structure of an anti-PEG antibody reveals an open ring that captures highly flexible PEG polymers. *Commun Chem*, 2020, vol. 3, 124
- **SHERMAN et al.** Role of the methoxy group in immune responses to mPEG-protein conjugates. *Bioconjug Chem*, 2012, vol. 23 (3), 485-499
- **SHERMAN et al.** Selectivity of binding of PEGs and PEG-like oligomers to anti-PEG antibodies induced by methoxyPEG-proteins. *Mol Immunol*, 2014, vol. 57 (2), 236-246
- **LIU et al.** Antibodies Predict Pegasparagase Allergic Reactions and Failure of Rechallenge. *J. Clin. Oncol*, 2019, vol. 37, 2051
- **ABU LILA et al.** Use of polyglycerol (PG), instead of polyethylene glycol (PEG), prevents induction of the accelerated blood clearance phenomenon against long-circulating liposomes upon repeated administration. *International Journal of Pharmaceutics*, 2013, vol. 456, 235-242
- **BENDELE, A.** ; **SEELY, J.** ; **RICHEY, C.** ; **SENNELLO, G.** ; **SHOPP, G**. Short Communication: Renal Tubular Vacuolation in Animals Treated with Polyethylene-Glycol-Conjugated Proteins. *Toxicol. Sei*, 1998, vol. 42, 152-157
- **FREY et al.** A Challenging Comonomer Pair: Copolymerization of Ethylene Oxide and Glycidyl Methyl Ether to Thermoresponsive Polyethers. *Macromolecules*, 2014, vol. 47, 5492-5500
- **FREY et al.** *Biomacromolecules*, 2014, vol. 15, 1935-1954
- **MACROMOL**. *Symp*, 2007, vol. 249-250, 392-397
- **BILLOUARD, C.** ; **CARLOTTI, S.** ; **DESBOIS, P.** ; **DEFFIEUX, A**. Controlled'' High-Speed Anionic Polymerization of Propylene Oxide Initiated by Alkali Metal Alkoxide/Trialkylaluminum Systems. *Macromolecules*, 2004, vol. 37 (11), 4038-4043
- **HERZBERGER, J.** ; **NIEDERER, K.** ; **POHLIT, H.** ; **SEIWERT, J.** ; **WORM, M.** ; **WURM, F. R.** ; **FREY, H**. Polymerization of Ethylene Oxide, Propylene Oxide, and Other Alkylene Oxides: Synthesis, Novel Polymer Architectures, and Bioconjugation. *Chemical reviews*, 2016, vol. 116 (4), 2170-2243
- **ISONO TAKUYA et al.** Design and synthesis of thermoresponsive aliphatic polyethers with a tunable phase transition temperature. *Polymer Chemistry*, 01 January 2017, vol. 8 (37), 5698-5707